# EUROPEAN PATENT APPLICATION

(11) **EP 4 668 779 A1**
(43) Date of publication of application: **24.12.2025**
(21) Application number: 24847526.1
(22) Date of filing: 02.02.2024
(51) Int. Cl.: H04R 25/00

(54) **HEARING LOSS COMPENSATION METHOD BASED ON HEARING AID DEVICE, AND TERMINAL**

(30) Priority: 02.08.2023 CN 202310972617
(71) Applicant: Huawei Technologies Co., Ltd., Shenzhen, Guangdong 518129 (CN)
(72) Inventor: QIN, Peng, Shenzhen, Guangdong 518129 (CN); YAO, Jingyu, Shenzhen, Guangdong 518129 (CN); MENG, Qinglin, Guangzhou, Guangdong 510641 (CN); ZHOU, Lei, Shenzhen, Guangdong 518129 (CN); KOU, Yiwei, Shenzhen, Guangdong 518129 (CN)
(74) Representative: Isarpatent
(86) International application number: PCT/CN2024/075477
(87) International publication number: WO 2025/025557

(57) **Abstract**

Embodiments of this application disclose a hearing-assistive device-based hearing loss compensation method and a terminal, to simplify a test procedure of hearing loss and improve accuracy of hearing loss compensation. The hearing-assistive device-based hearing loss compensation method is performed by the terminal. A communication connection is established between the terminal and a hearing-assistive device to be fitted. The method includes: performing a hearing loss test on a hearing-assistive object to obtain a hearing loss test result; performing hearing loss compensation based on the hearing loss test result to obtain a hearing loss compensation gain; and sending the hearing loss compensation gain to the hearing-assistive device, where the hearing-assistive device is configured to perform hearing loss compensation on a first speech signal based on the hearing loss compensation gain to obtain a second speech signal.

## Description

This application claims priority to Chinese Patent Application No. 202310972617.9, filed with the China National Intellectual Property Administration on August 2, 2023 and entitled "HEARING-ASSISTIVE DEVICE-BASED HEARING LOSS COMPENSATION METHOD AND TERMINAL", which is incorporated herein by reference in its entirety.

### TECHNICAL FIELD

This application relates to the field of computer technologies, and in particular, to a hearing-assistive device-based hearing loss compensation method and a terminal.

### BACKGROUND

Hearing loss is an auditory disorder, and means partial or complete loss of hearing, which may occur in one ear or both ears. There are four levels of hearing loss: 26 dB to 40 dB: light; 41 dB to 60 dB: medium; 61 dB to 80 dB: heavy; greater than 80 dB: extremely heavy.

Users with hearing loss need to use hearing-assistive products such as hearing aids to implement assistive listening. Audiometry is performed on the users with hearing loss in a fitting center, professional hearing aid dispensers select hearing aids, and then repeated manual fine-tuning and testing are performed, to complete an entire fitting process.

The foregoing solution has problems of low accuracy of manual tests and a complex test process.

### SUMMARY

Embodiments of this application provide a hearing-assistive device-based hearing loss compensation method and a terminal, to simplify a test procedure of hearing loss and improve accuracy of hearing loss compensation.

To resolve the foregoing technical problem, embodiments of this application provide the following technical solutions.

According to a first aspect, an embodiment of this application provides a hearing-assistive device-based hearing loss compensation method. The method is performed by a terminal. A communication connection is established between the terminal and a hearing-assistive device to be fitted. The method includes: performing a hearing loss test on a hearing-assistive object to obtain a hearing loss test result; performing hearing loss compensation based on the hearing loss test result to obtain a hearing loss compensation gain; and sending the hearing loss compensation gain to the hearing-assistive device, where the hearing-assistive device is configured to perform hearing loss compensation on a first speech signal based on the hearing loss compensation gain to obtain a second speech signal.

In the foregoing solution, the terminal may perform hearing loss compensation on the hearing-assistive object wearing the hearing-assistive device, to implement fitting on the hearing-assistive device of the hearing-assistive object. An entire process of hearing loss compensation may be implemented by interaction between the terminal and the hearing-assistive device, and no manual test needs to be performed by a doctor. Therefore, a hearing loss test procedure can be quickly and conveniently performed, and the terminal interacts with the hearing-assistive device worn by the hearing-assistive object, to complete hearing loss compensation. Hearing loss compensation can be performed on the hearing-assistive object wearing the hearing-assistive device, so that accuracy of hearing loss compensation can be improved.

In a possible implementation of the first aspect, performing the hearing loss test on the hearing-assistive object to obtain the hearing loss test result includes: performing hearing detection on the hearing-assistive object to obtain a feedback speed of the hearing-assistive object; generating a first test signal based on the feedback speed and a preset first volume, and sending the first test signal to the hearing-assistive device; and obtaining the hearing loss test result based on the first test signal and a first feedback instruction, where the first feedback instruction includes: a feedback instruction detected by the terminal when the hearing-assistive device plays the first test signal. In the foregoing solution, the feedback speed of the hearing-assistive object may be obtained by performing hearing detection on the hearing-assistive object, and the first test signal is generated based on the feedback speed. The first test signal may be used to obtain the hearing loss test result. The first test signal sent based on the feedback speed of the hearing-assistive object can implement an accurate hearing loss test on the hearing-assistive object, to improve accuracy of the hearing loss test result.

In a possible implementation of the first aspect, performing hearing detection on the hearing-assistive object to obtain the feedback speed of the hearing-assistive object includes: sending a second test signal to the hearing-assistive device; and obtaining the feedback speed based on the second test signal and a second feedback instruction, where the second feedback instruction includes: a feedback instruction detected by the terminal when the hearing-assistive device plays the second test signal. In the foregoing solution, the hearing-assistive object may tap a feedback button in a UI of the terminal, so that the terminal detects the second feedback instruction sent by the hearing-assistive object, and the terminal obtains the feedback speed based on the second test signal and the second feedback instruction.

In a possible implementation of the first aspect, generating the first test signal based on the feedback speed and the preset first volume includes: generating the first test signal with a random length based on the feedback speed and the preset first volume, where the first test signal includes test signals at a plurality of frequencies used to measure a hearing loss level of the hearing-assistive object. In the foregoing solution, the terminal generates, based on the first volume and the feedback speed, a test signal with a random length within a preset length range, to avoid inertia fatigue of the hearing-assistive object and improve accuracy of a hearing loss level test.

In a possible implementation of the first aspect, the first test signal includes a random mute signal; and the method further includes: when the hearing-assistive device plays the random mute signal, detecting a misoperation feedback instruction sent by the hearing-assistive object; and sending a misoperation prompt signal to the hearing-assistive device based on the misoperation feedback instruction. In the foregoing solution, the random mute signal is added to the first test signal. When a user taps a button during playing of the mute signal, the terminal may send the misoperation prompt signal to the hearing-assistive device, and the hearing-assistive device plays the misoperation prompt signal, to prompt the user to correctly perform a tap, thereby avoiding inertia fatigue of the user, reducing a misoperation, and improving a tap accuracy rate of the user.

In a possible implementation of the first aspect, generating the first test signal based on the feedback speed and the preset first volume, and sending the first test signal to the hearing-assistive device includes: generating, based on the feedback speed, the preset first volume, and a preset volume increase/decrease difference, a plurality of first test signals corresponding to a first frequency, and sending the plurality of first test signals to the hearing-assistive device, where a volume of one first test signal in the plurality of first test signals is the first volume, and a volume of a first test signal other than the one first test signal in the plurality of first test signals is determined based on the first volume and the volume increase/decrease difference; and obtaining the hearing loss test result based on the first test signal and the first feedback instruction includes: determining a first flip point based on a plurality of first feedback instructions corresponding to the plurality of first test signals, where the first flip point includes a time point at which two adjacent first feedback instructions in the plurality of first feedback instructions indicate different listening results; obtaining a second flip point adjacent to the first flip point; and when a difference between the first flip point and the second flip point is greater than a preset sound pressure threshold, clearing a frequency test result corresponding to the first flip point; or when a difference between the first flip point and the second flip point is less than or equal to a preset sound pressure threshold, obtaining the hearing loss test result based on a frequency test result of the first flip point. In the foregoing solution, the terminal may record the difference between the first flip point and the second flip point that are adjacent to each other. If the difference is less than or equal to the preset sound pressure threshold, it indicates that the currently recorded frequency test result of the first flip point is accurate, and a frequency test may continue to be performed. The frequency test result of the first flip point may be used to obtain the hearing loss test result.

In a possible implementation of the first aspect, obtaining the hearing loss test result based on the frequency test result of the first flip point includes: when a quantity of times that the frequency test result reaches an extreme value exceeds a times threshold, and/or a quantity of first test signals sent at the first frequency exceeds a quantity threshold, determining the hearing loss test result based on the frequency test result of the first flip point. In the foregoing solution, the terminal may further set a frequency test end condition. When the frequency test result meets the preset frequency test end condition, the terminal stops testing the first frequency, and determines the hearing loss test result based on the frequency test result of the first flip point. The frequency is tested to improve efficiency of the hearing loss test.

In a possible implementation of the first aspect, performing hearing loss compensation based on the hearing loss test result to obtain the hearing loss compensation gain includes: performing real-time compensation processing in time domain and/or frequency domain based on the hearing loss test result to obtain a first compensation gain; and performing speech correction on the first compensation gain based on a preset speech test signal to obtain the hearing loss compensation gain. In the foregoing solution, after the first compensation gain is determined, the terminal may send the preset speech test signal to the hearing-assistive device, and perform speech correction on the first compensation gain based on the speech test signal, to obtain the hearing loss compensation gain. The foregoing speech correction can improve a speech recognition rate.

In a possible implementation of the first aspect, performing real-time compensation processing in time domain and/or frequency domain based on the hearing loss test result to obtain the first compensation gain includes: obtaining a first hearing loss value at the first frequency in the hearing loss test result; determining, based on a mapping relationship between a hearing loss value and a correction gain value, a first correction gain value corresponding to the first hearing loss value in time domain and/or frequency domain; and determining the first compensation gain based on the first compensation gain value. In the foregoing solution, real-time compensation may be performed based on the mapping relationship between the hearing loss value and the correction gain value, to improve accuracy of hearing loss compensation.

In a possible implementation of the first aspect, performing real-time compensation processing in time domain and/or frequency domain based on the hearing loss test result to obtain the first compensation gain further includes: determining a hearing curve based on the hearing loss test result; and determining the mapping relationship between the hearing loss value and the correction gain value based on a classification result corresponding to the hearing curve. In the foregoing solution, the hearing loss test result may include one or more hearing curves. The terminal may obtain the hearing curve, classify a type of the hearing curve based on a predetermined hearing curve library, to obtain the classification result corresponding to the hearing curve, and select, based on the classification result, the corresponding mapping relationship between the hearing loss value and the correction gain value, so that the mapping relationship between the hearing loss value and the correction gain value can be used for real-time compensation.

In a possible implementation of the first aspect, determining, based on the mapping relationship between the hearing loss value and the correction gain value, the first correction gain value corresponding to the first hearing loss value in time domain and/or frequency domain includes: dividing, based on an octave, an external input signal collected by the hearing-assistive device into a plurality of subbands; obtaining speech estimation signals and noise estimation signals corresponding to the plurality of subbands; performing speech intelligibility index analysis based on the speech estimation signals and the noise estimation signals to obtain loudness of hearing loss; determining a first frequency domain compensation gain of the first hearing loss value in frequency domain based on the mapping relationship between the hearing loss value and the correction gain value; and performing iterative optimization on the first frequency domain compensation gain based on the speech estimation signals and the noise estimation signals corresponding to the plurality of subbands and the loudness of the hearing loss, to obtain a second frequency domain compensation gain that maximizes intelligibility within preset loudness, where the second frequency domain compensation gain belongs to the first correction gain value. In the foregoing solution, accuracy of hearing loss compensation can be improved by using the foregoing hearing loss compensation in frequency domain.

In a possible implementation of the first aspect, determining, based on the mapping relationship between the hearing loss value and the correction gain value, the first correction gain value corresponding to the first hearing loss value in time domain and/or frequency domain includes: determining a first time domain compensation gain of the first hearing loss value in time domain based on the mapping relationship between the hearing loss value and the correction gain value; extracting envelope information of an external input signal collected by the hearing-assistive device; and when an average value of the envelope information is greater than a preset envelope threshold, performing linear attenuation processing on the first time domain compensation gain to obtain a second time domain compensation gain, where the second time domain compensation gain belongs to the first correction gain value; or when an average value of the envelope information is less than or equal to a preset envelope threshold, performing linear improvement processing on the first time domain compensation gain to obtain a third time domain compensation gain, where the third time domain compensation gain belongs to the first correction gain value. In the foregoing solution, the terminal performs linear attenuation processing on the first time domain compensation gain, obtains the second time domain compensation gain through the linear attenuation processing, and determines the first correction gain value based on the second time domain compensation gain, so that linear attenuation occurs on an envelope of the external input signal, to improve accuracy of hearing loss compensation. The terminal performs linear improvement processing on the first time domain compensation gain, obtains the third time domain compensation gain through the linear improvement processing, and determines the first correction gain value based on the third time domain compensation gain, so that linear improvement occurs on an envelope of the external input signal, to improve accuracy of hearing loss compensation.

In a possible implementation of the first aspect, performing speech correction on the first compensation gain based on the preset speech test signal to obtain the hearing loss compensation gain includes: generating a speech test signal based on an audio spectral centroid in a frequency band division manner, where the speech test signal includes test words at a plurality of frequencies corresponding to different audio spectral centroids; sending the speech test signal to the hearing-assistive device; and performing speech correction on the first compensation gain based on the speech test signal and a third feedback instruction to obtain the hearing loss compensation gain, where the third feedback instruction includes a feedback instruction detected by the terminal when the hearing-assistive device plays a third test signal. In the foregoing solution, the terminal performs speech correction on the first compensation gain based on the speech test signal and the third feedback instruction to obtain the hearing loss compensation gain. The speech test signal is generated in the foregoing multiple-alternative forced-choice manner, and the speech test signal is used for speech correction, so that accuracy of hearing loss compensation can be improved.

In a possible implementation of the first aspect, the method further includes: detecting an ambient noise of the hearing-assistive device, and when the ambient noise is greater than or equal to a preset noise threshold, sending a noise prompt signal to the hearing-assistive device; or when the ambient noise is less than a preset noise threshold, triggering execution of the following step: performing the hearing loss test on the hearing-assistive object to obtain the hearing loss test result. In the foregoing solution, the terminal may first detect an environment in which the hearing-assistive device is located, to ensure that a subsequent hearing loss compensation procedure can be correctly performed, and improve accuracy of hearing loss compensation.

In a possible implementation of the first aspect, performing speech correction on the first compensation gain based on the preset speech test signal includes: obtaining the speech test signal from a preset corpus, where the corpus includes a plurality of groups of speech test signals, each group of speech test signals includes initial consonant-based single-character groups divided into a same group based on an audio spectral centroid and different octaves, and one initial consonant-based single-character group includes two or more randomly extracted confusable words; playing the two or more confusable words in the same initial consonant-based single-character group in a random order through the hearing-assistive device; detecting a fourth feedback instruction sent by the hearing-assistive object; obtaining a consistency error frequency band of the hearing-assistive object based on the fourth feedback instruction and the speech test signal; and adjusting the first compensation gain based on the consistency error frequency band. In the foregoing solution, each group of words is randomly extracted and played in the random order, so that a problem of speech material reuse effectiveness can be resolved. The consistency error frequency band can be determined based on the fourth feedback instruction, to meet differentiated requirements of different users for volumes, timbre equalization, noise reduction, and the like. The speech test signal is generated, and the speech test signal is used for speech correction, so that accuracy of hearing loss compensation can be improved.

In a possible implementation of the first aspect, determining, based on the mapping relationship between the hearing loss value and the correction gain value, the first correction gain value corresponding to the first hearing loss value in time domain and/or frequency domain further includes: performing human voice separation on the external input signal collected by the hearing-assistive device, to obtain a human voice signal; and performing wide dynamic range compression compensation and envelope enhancement on the human voice signal to obtain a compensated and enhanced human voice signal; and dividing, based on the octave, the external input signal collected by the hearing-assistive device into the plurality of subbands includes: dividing the compensated and enhanced human voice signal into a plurality of subbands based on the octave. In the foregoing solution, wide dynamic range compression compensation and envelope enhancement are performed on the human voice signal to obtain the compensated and enhanced human voice signal. The human voice signal may be used for subsequent compensation gain calculation, so that a better human voice compensation effect can be provided, and the speech recognition rate can be improved.

According to a second aspect, an embodiment of this application further provides a terminal. A communication connection is established between the terminal and a hearing-assistive device to be fitted, and the terminal includes:
a test module, configured to perform a hearing loss test on a hearing-assistive object to obtain a hearing loss test result;
a compensation module, configured to perform hearing loss compensation based on the hearing loss test result to obtain a hearing loss compensation gain; and
a sending module, configured to send the hearing loss compensation gain to the hearing-assistive device, where the hearing-assistive device is configured to perform hearing loss compensation on a first speech signal based on the hearing loss compensation gain to obtain a second speech signal.

In the foregoing solution, the terminal may perform hearing loss compensation on the hearing-assistive object wearing the hearing-assistive device, to implement fitting on the hearing-assistive device of the hearing-assistive object. An entire process of hearing loss compensation may be implemented by interaction between the terminal and the hearing-assistive device, and no manual test needs to be performed by a doctor. Therefore, a hearing loss test procedure can be quickly and conveniently performed, and the terminal interacts with the hearing-assistive device worn by the hearing-assistive object, to complete hearing loss compensation. Hearing loss compensation can be performed on the hearing-assistive object wearing the hearing-assistive device, so that accuracy of hearing loss compensation can be improved.

In the second aspect of this application, composition modules of the terminal may further perform the steps described in the first aspect and the possible implementations. For details, refer to the descriptions in the first aspect and the possible implementations.

According to a third aspect, an embodiment of this application further provides a terminal. The terminal includes a processor and a memory, and the processor and the memory communicate with each other;
the memory is configured to store instructions; and
the processor is configured to execute the instructions in the memory, to perform the method in the first aspect.

According to a fourth aspect, an embodiment of this application provides a computer-readable storage medium. The computer-readable storage medium stores instructions. When the computer-readable storage medium runs on a computer, the computer is enabled to perform the method in the first aspect.

According to a fifth aspect, an embodiment of this application provides a computer program product including instructions. When the computer program product runs on a computer, the computer is enabled to perform the method in the first aspect.

According to a sixth aspect, an embodiment of this application provides a communication apparatus. The communication apparatus may include an entity such as a terminal device or a chip. The communication apparatus includes a processor and a memory. The memory is configured to store instructions. The processor is configured to execute the instructions in the memory, so that the communication apparatus performs the method in the first aspect.

According to a seventh aspect, this application provides a chip system. The chip system includes a processor, configured to support a terminal in implementing functions in the foregoing aspects, for example, sending or processing data and/or information in the foregoing methods. In a possible design, the chip system further includes a memory. The memory is configured to store program instructions and data that are necessary for the terminal. The chip system may include a chip, or may include a chip and another discrete component.

### BRIEF DESCRIPTION OF DRAWINGS

FIG. 1 is a diagram of a composition architecture of a hearing loss compensation system according to an embodiment of this application;
FIG. 2 is an interaction diagram of a hearing loss compensation method according to an embodiment of this application;
FIG. 3 is a schematic block flowchart of a hearing loss compensation method according to an embodiment of this application;
FIG. 4 is a diagram of an application framework of a hearing loss compensation system according to an embodiment of this application;
FIG. 5A to FIG. 5E are a diagram of a UI of a terminal in a hearing loss compensation system according to an embodiment of this application;
FIG. 6 is a schematic flowchart of an application scenario of a hearing loss compensation method according to an embodiment of this application;
FIG. 7A and FIG. 7B are a schematic flowchart of an audiometry algorithm according to an embodiment of this application;
FIG. 8a is a diagram of an audiometry configuration UI according to an embodiment of this application;
FIG. 8b is a diagram of a test guidance UI according to an embodiment of this application;
FIG. 8c is a diagram of a reaction speed test UI according to an embodiment of this application;
FIG. 8d is a diagram of a hearing test UI according to an embodiment of this application;
FIG. 8e is a diagram of a speech test UI according to an embodiment of this application;
FIG. 8f is a diagram of a feedback adjustment UI according to an embodiment of this application;
FIG. 9A to FIG. 9C are a schematic flowchart of a compensation algorithm according to an embodiment of this application;
FIG. 10A and FIG. 10B are a schematic flowchart of speech correction according to an embodiment of this application;
FIG. 11 is a schematic flowchart of an application scenario of a hearing loss compensation method according to an embodiment of this application;
FIG. 12 is a diagram of a composition structure of a terminal according to an embodiment of this application; and
FIG. 13 is a diagram of a composition structure of a terminal according to an embodiment of this application.

### DESCRIPTION OF EMBODIMENTS

The following describes embodiments of this application with reference to the accompanying drawings.

In the specification, claims, and the accompanying drawings of this application, the terms "first", "second", and the like are intended to distinguish between similar objects but do not necessarily indicate a specific order or sequence. It should be understood that the terms used in such a way are interchangeable in proper circumstances, which is merely a discrimination manner that is used when objects having a same attribute are described in embodiments of this application. In addition, the terms "include", "contain" and any other variants mean to cover the non-exclusive inclusion, so that a process, method, system, product, or device that includes a series of units is not necessarily limited to those units, but may include other units not expressly listed or inherent to such a process, method, product, or device.

The technical solutions in embodiments of this application may be applied to various hearing loss compensation systems. The hearing loss compensation system may implement automatic fitting on a hearing-assistive device of a hearing-assistive object. The hearing-assistive object is an object that needs to use the hearing-assistive device. For example, the hearing-assistive object is a human ear of a user, and the hearing-assistive device is an audio signal playback terminal. For example, the hearing-assistive device may be a Bluetooth headset, a wired headset, or a hearing aid. The hearing-assistive device may also be referred to as a hearing-assistive component. The user may receive, by wearing the hearing-assistive device, a speech signal sent by the terminal, where the speech signal is a speech signal obtained after the terminal performs hearing loss compensation on the hearing-assistive object. The hearing loss compensation system provided in embodiments of this application does not require a doctor to perform a manual test, so that a hearing loss test procedure can be simplified, and accuracy of hearing loss compensation can be improved.

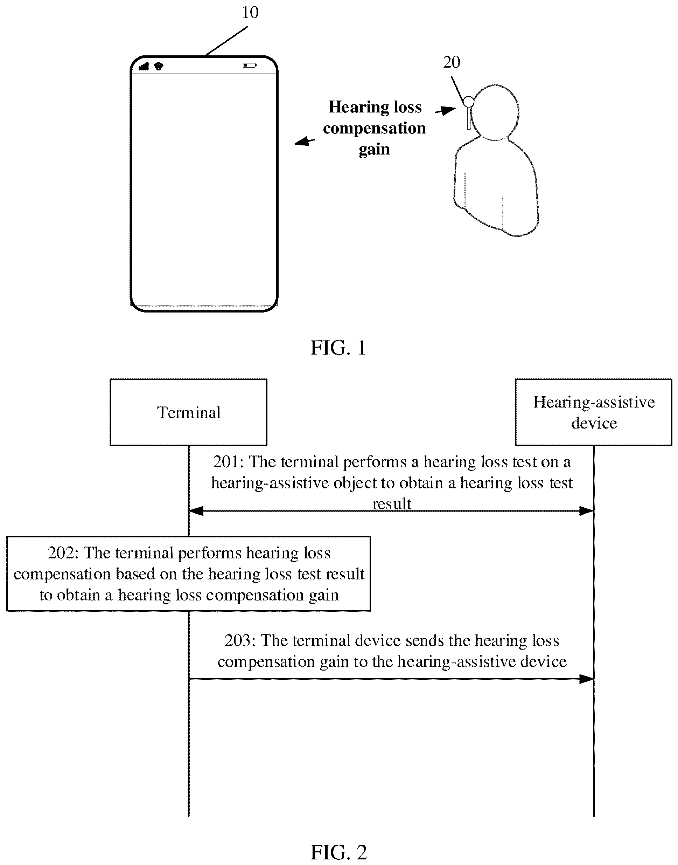

FIG. 1 shows a hearing loss compensation system according to an embodiment of this application, including a terminal 10 and a hearing-assistive device 20 to be fitted. A communication connection is established between the terminal 10 and the hearing-assistive device 20. For example, the terminal 10 communicates with the hearing-assistive device 20 by using a wireless network. The terminal 10 may perform hearing loss compensation on a hearing-assistive object wearing the hearing-assistive device 20, and does not require a doctor to perform a manual test. Therefore, a hearing loss test procedure can be quickly and conveniently performed. The terminal 10 interacts with the hearing-assistive device 20 worn by the hearing-assistive object, so that accuracy of hearing loss compensation can be improved.

In this embodiment of this application, the hearing loss compensation system uses an autonomous fitting architecture combining software and hardware. Specifically, the hearing loss compensation system includes a terminal and a hearing-assistive device. The terminal is an operating software carrier terminal, for example, the terminal includes a mobile phone, a tablet computer, a personal computer, and the like. The hearing-assistive device is an audio signal playback terminal, for example, the hearing-assistive device is a Bluetooth headset, a wired headset, a hearing aid, and the like. The terminal may specifically perform a hearing loss test and hearing loss compensation. The terminal performs hearing loss compensation on an original speech signal to obtain a target speech signal, and then sends the target speech signal to the hearing-assistive device. After receiving the target speech signal, the hearing-assistive device plays the target speech signal through an audio play port, so that the hearing-assistive object hears the target speech signal obtained after the hearing loss compensation, to implement automatic fitting on the hearing-assistive device of the hearing-assistive object.

The following describes the terminal in embodiments of this application. The terminal may also be referred to as a terminal device. The terminal device may include at least one of the following: user equipment (user equipment, UE), a mobile station (mobile station, MS), a mobile terminal (mobile terminal, MT), a terminal, a terminal apparatus, and the like. The terminal device is a device that provides voice and/or data connectivity for a user, or a chip disposed in the device, for example, a handheld device or a vehicle-mounted device having a wireless connection function. Currently, some examples of the terminal device are a mobile phone (mobile phone), a tablet computer, a laptop computer, a palmtop computer, a mobile internet device (mobile internet device, MID), a wearable device, a virtual reality (virtual reality, VR) device, an augmented reality (augmented reality, AR) device, a wireless terminal in industrial control (industrial control), a wireless terminal in self driving (self driving), a wireless terminal in remote medical surgery (remote medical surgery), a wireless terminal in a smart grid (smart grid), a wireless terminal in transportation safety (transportation safety), a wireless terminal in a smart city (smart city), a wireless terminal in a smart home (smart home), or the like. For example, the terminal device may be a vehicle-mounted communication module or another embedded communication module, or may be a handheld communication device of the user, including a mobile phone, a tablet computer, or the like.

The terminal included in the hearing loss compensation system provided in this embodiment of this application may perform a hearing-assistive device-based hearing loss compensation method. The user does not need to go to a hearing center to search for a professional hearing aid dispenser for fitting, and can independently complete an entire fitting process, and achieve a good hearing-assistive effect. The following describes the hearing loss compensation method performed by the terminal by using an example. A communication connection is established between the terminal and a hearing-assistive device to be fitted. Refer to FIG. 2. The method mainly includes the following steps.

201: The terminal performs a hearing loss test on a hearing-assistive object to obtain a hearing loss test result.

The communication connection is established between the terminal and the hearing-assistive device. For example, a Bluetooth connection is established between the terminal and the hearing-assistive device. After the hearing-assistive object wears the hearing-assistive device, the terminal device first performs the hearing loss test on the hearing-assistive object, for example, may test hearing loss of a left ear and a right ear of the hearing-assistive object, to obtain the hearing loss test result, where the hearing loss test result may also be referred to as a hearing impairment test result. For example, the hearing loss test result may include a left-ear hearing loss test result of the hearing-assistive object and a right-ear test result of the hearing-assistive object. Specifically, the hearing loss test result may include a parameter value of the hearing loss test, and the parameter value of the hearing loss test may be recorded in a manner such as a set, a list, or a curve in the hearing loss test result.

In this embodiment of this application, the terminal performs the hearing loss test on the hearing-assistive object without requiring a manual operation of a doctor, and the terminal interacts with the hearing-assistive device to implement the test.

Specifically, there are a plurality of manners of the hearing loss test. This is not limited in this embodiment of this application. For details, refer to examples in subsequent embodiments for description.

In some embodiments of this application, in addition to the foregoing step of the hearing loss compensation method, the hearing loss compensation method performed by the terminal further includes the following step.

A1: The terminal determines, based on a sound signal collected by the hearing-assistive device, that the hearing-assistive device does not meet a wearing requirement, and sends a wearing prompt signal to the hearing-assistive device.

After the hearing-assistive object wears the hearing-assistive device, the terminal detects wearing airtightness. Specifically, the terminal determines, based on the sound signal collected by the hearing-assistive device, whether the hearing-assistive device meets the wearing requirement, and sends the wearing prompt signal to the hearing-assistive device when the hearing-assistive device does not meet the wearing requirement, so that the hearing-assistive object can adjust wearing of the hearing-assistive device, to ensure that a subsequent hearing loss compensation procedure can be correctly performed, and improve accuracy of hearing loss compensation.

For example, the terminal may detect a feedforward (FeedForward, FF) signal or a feedback (FeedBack, FB) signal based on a prompt tone, to detect wearing consistency. If wearing is good, a subsequent hearing compensation procedure is performed; otherwise, a wearing prompt tone is played.

In some embodiments of this application, in addition to the foregoing step of the hearing loss compensation method, the hearing loss compensation method performed by the terminal further includes the following step.

B1: The terminal detects an ambient noise of the hearing-assistive device, and when the ambient noise is greater than or equal to a preset noise threshold, the terminal sends a noise prompt signal to the hearing-assistive device.

When an ambient noise is less than a preset noise threshold, execution of the foregoing step 201 is triggered: The terminal performs the hearing loss test on the hearing-assistive object to obtain the hearing loss test result.

After the hearing-assistive object wears the hearing-assistive device, the terminal may further detect the ambient noise of the hearing-assistive device, where the ambient noise may also be referred to as an ambient background noise, to determine whether a wearing manner of the hearing-assistive device needs to be adjusted to perform a subsequent procedure. Specifically, when the ambient noise is greater than or equal to the preset noise threshold, the terminal sends the noise prompt signal to the hearing-assistive device, so that the hearing-assistive object adjusts an environment in which the hearing-assistive device is located. When the ambient noise is less than the preset noise threshold, an environment in which the hearing-assistive device is located is suitable for performing the hearing loss test, and the terminal triggers execution of the foregoing step 201. The terminal may first detect the environment in which the hearing-assistive device is located, to ensure that the subsequent hearing loss compensation procedure can be correctly performed, and improve accuracy of hearing loss compensation.

202: The terminal performs hearing loss compensation based on the hearing loss test result to obtain a hearing loss compensation gain.

After the terminal obtains the hearing loss test result, the terminal may determine hearing loss of the tested hearing-assistive object, so that the terminal device needs to perform hearing loss compensation on the hearing-assistive object. The terminal determines the hearing loss compensation gain based on the hearing loss test result. The hearing loss compensation gain may include a gain value of hearing loss compensation that needs to be performed on the hearing-assistive object. For example, the hearing loss compensation gain may specifically include a frequency domain compensation gain, the hearing loss compensation gain may specifically include a time domain compensation gain, or the hearing loss compensation gain may specifically include a frequency domain compensation gain and a time domain compensation gain. This is not limited herein.

203: The terminal device sends the hearing loss compensation gain to the hearing-assistive device, where the hearing-assistive device is configured to perform hearing loss compensation on a first speech signal based on the hearing loss compensation gain to obtain a second speech signal.

After the terminal obtains the hearing loss compensation gain, the terminal sends the hearing loss compensation gain through the communication connection to the hearing-assistive device, and the hearing-assistive device receives and stores the hearing loss compensation gain. For example, the hearing-assistive device may be specifically a headset. The headset includes a digital signal processor (digital signal processor, DSP). The DSP includes a wide dynamic range control (wide dynamic ratio control, WDRC) module and an envelope modulation module. The WDRC module and the envelope modulation module may perform amplification processing on a speech signal based on the hearing loss compensation gain.

The first speech signal is an original audio signal that needs to be played to the hearing-assistive object. For example, the first speech signal may be call voice data, or audio channel data included in video data, or the first speech signal may be speech data, sentence data, music data, or the like. The hearing-assistive device obtains the to-be-transmitted first speech signal. For example, the hearing-assistive device may obtain first speech data from the terminal or a cloud server, or the hearing-assistive device may receive first speech data from another terminal. The hearing-assistive device obtains the first speech signal, and performs hearing loss compensation on the first speech signal based on the hearing loss compensation gain determined in the foregoing steps 201 and 202, to obtain the second speech signal. For example, the second speech signal may include at least one of the following: speech data, sentence data, music data, and the like that are obtained after hearing loss compensation is performed based on the hearing loss compensation gain.

Specifically, that the terminal device sends the hearing loss compensation gain to the hearing-assistive device in 203 includes:
The terminal performs hearing loss compensation on the to-be-transmitted first speech signal based on the hearing loss compensation gain to obtain the second speech signal, and sending the second speech signal to the hearing-assistive device.

The terminal may perform hearing loss compensation on the first speech signal, and then the hearing-assistive device sends the second speech signal obtained after the hearing loss compensation, so that the hearing-assistive device receives the second speech signal obtained after the hearing loss compensation, and the hearing-assistive device needs to play only the second speech signal, so that a processing procedure of the hearing-assistive device can be simplified.

It can be learned from the example descriptions of the foregoing embodiment that, in this embodiment of this application, the terminal may perform hearing loss compensation on the hearing-assistive object wearing the hearing-assistive device, to implement fitting on the hearing-assistive device of the hearing-assistive object. An entire process of hearing loss compensation may be implemented by interaction between the terminal and the hearing-assistive device, and no manual test needs to be performed by a doctor. Therefore, a hearing loss test procedure can be quickly and conveniently performed, and the terminal interacts with the hearing-assistive device worn by the hearing-assistive object, to complete hearing loss compensation. Hearing loss compensation can be performed on the hearing-assistive object wearing the hearing-assistive device, so that accuracy of hearing loss compensation can be improved.

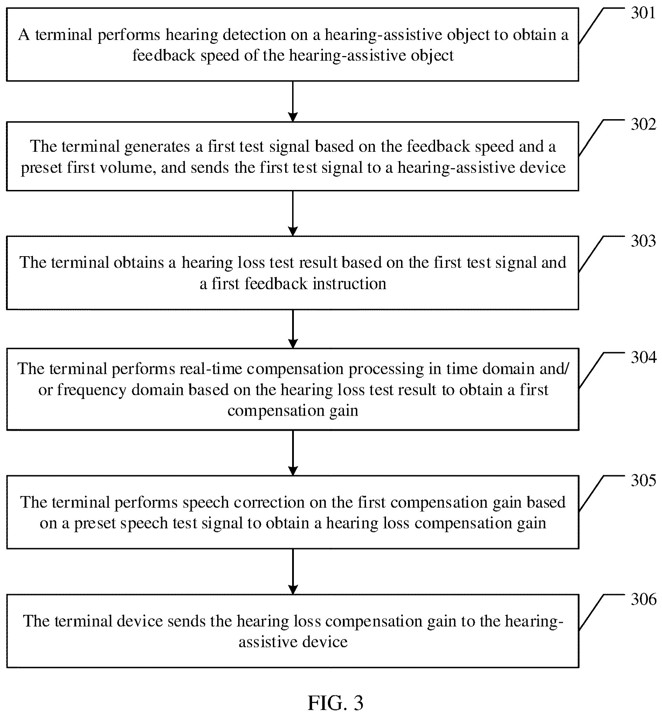

FIG. 3 shows another hearing loss compensation method according to an embodiment of this application. The method mainly includes the following steps.

301: A terminal performs hearing detection on a hearing-assistive object to obtain a feedback speed of the hearing-assistive object.

The terminal may first detect a hearing level of the hearing-assistive object. Specifically, the terminal performs hearing detection on the hearing-assistive object to perform a reaction speed test, to obtain the feedback speed of the hearing-assistive object. The feedback speed may also be referred to as a reaction speed. For example, the feedback speed may be time required by the hearing-assistive object to respond to a speech signal after the speech signal is heard, and the feedback speed may be used to generate a test signal. The feedback speed may separately include a feedback speed of a left ear of the hearing-assistive object and a feedback speed of a right ear of the hearing-assistive object.

302: The terminal generates a first test signal based on the feedback speed and a preset first volume, and sends the first test signal to a hearing-assistive device.

After the terminal obtains the feedback speed of the hearing-assistive object, the terminal generates the first test signal based on the preset first volume and the feedback speed, where the first test signal may be a speech signal used to test hearing loss of the hearing-assistive object, and the first test signal may be one or more first test signals. The first volume may be an initial audio signal volume. After generating the first test signal, the terminal sends the first test signal through a communication connection between the terminal and the hearing-assistive device.

303: The terminal obtains a hearing loss test result based on the first test signal and a first feedback instruction, where the first feedback instruction includes a feedback instruction detected by the terminal when the hearing-assistive device plays the first test signal.

After the hearing-assistive device receives the first test signal, the hearing-assistive device plays the first test signal to the hearing-assistive object. If the hearing-assistive object hears audio content of the first test signal, the hearing-assistive object may send the first feedback instruction to the terminal. For example, the hearing-assistive object taps a feedback button in a UI, so that the terminal detects the first feedback instruction. The accurate hearing loss test can be performed on the hearing-assistive object, and accuracy of the hearing loss test result can be improved. The terminal obtains the hearing loss test result based on the first test signal and the first feedback instruction. For example, the hearing loss test result may include left and right ear audiometry curves of the hearing-assistive object.

In some embodiments of this application, that the terminal performs hearing detection on the hearing-assistive object to obtain the feedback speed of the hearing-assistive object in 301 includes:
C1: The terminal sends a second test signal to the hearing-assistive device.

C2: The terminal obtains a feedback speed based on the second test signal and a second feedback instruction, where the second feedback instruction includes a feedback instruction detected by the terminal when the hearing-assistive device plays the second test signal.

The terminal sends the second test signal to the hearing-assistive device, and the hearing-assistive device plays the second test signal, where the second test signal includes a tap speed test audio. If the hearing-assistive object hears audio content of the second test signal, the hearing-assistive object may send the second feedback instruction to the terminal. For example, the hearing-assistive object may tap a feedback button in a UI of the terminal, so that the terminal detects the second feedback instruction sent by the hearing-assistive object. The terminal obtains the feedback speed based on the second test signal and the second feedback instruction. Specifically, the terminal records time from starting to play the second test signal to sending the second feedback instruction by the hearing-assistive object, to obtain the feedback speed of the hearing-assistive object.

In some embodiments of this application, that the terminal generates the first test signal based on the feedback speed and the preset first volume in 302 includes:
D1: The terminal generates the first test signal with a random length based on the feedback speed and the preset first volume, where the first test signal includes test signals at a plurality of frequencies used to measure a hearing loss level of the hearing-assistive object.

The terminal generates, based on the first volume and the feedback speed, a test signal with a random length within a preset length range, to avoid inertia fatigue of the hearing-assistive object. The hearing-assistive object may select monaural or binaural audiometry, for example, test a plurality of frequencies. The first test signal includes the test signals at the plurality of frequencies, and the plurality of frequencies include a key pure tone audiometry (Pure Tone Audiometry, PTA) frequency for measuring a hearing loss level of a user. Therefore, inertia fatigue of the hearing-assistive object can be avoided, and accuracy of the hearing loss level test can be improved.

In some embodiments of this application, the first test signal includes a random mute signal, and the random mute signal is an audio signal including a randomly generated mute frequency. In addition to the foregoing step of the hearing loss compensation method, the hearing loss compensation method performed by the terminal further includes the following step.

E1: When the hearing-assistive device plays the random mute signal, the terminal detects a misoperation feedback instruction sent by the hearing-assistive object.

The first test signal may include the random mute signal. When the hearing-assistive device plays the random mute signal, the terminal detects the misoperation feedback instruction sent by the hearing-assistive object. When the random mute signal is played, if the hearing-assistive object does not send the misoperation feedback instruction, it indicates that the hearing-assistive object can correctly identify mute. When the random mute signal is played, if the hearing-assistive object sends the misoperation feedback instruction to the terminal, the terminal may detect the misoperation feedback instruction sent by the hearing-assistive object.

E2: The terminal sends a misoperation prompt signal to the hearing-assistive device based on the misoperation feedback instruction.

When the hearing-assistive device plays the random mute signal, if the terminal detects the misoperation feedback instruction sent by the hearing-assistive object, it indicates that the hearing-assistive object generates a misoperation at this time, and the terminal may send the misoperation prompt signal to the hearing-assistive device, to prompt the hearing-assistive object of the generated misoperation. In some possible implementations of this application, an inertia operation is easily generated when the user continuously taps a button in the UI of the terminal. In this embodiment of this application, the random mute signal is added to the first test signal. When the user taps the button during playing of the mute signal, the terminal may send the misoperation prompt signal to the hearing-assistive device, and the hearing-assistive device plays the misoperation prompt signal, to prompt the user to correctly perform a tap, thereby avoiding inertia fatigue of the user, reducing a misoperation, and improving a tap accuracy rate of the user.

In some embodiments of this application, the first test signal generated by the terminal may be a plurality of first test signals. For the plurality of first test signals, the terminal may determine a flip point, and perform determining on the flip point, to determine whether to clear the flip point, thereby improving audiometry accuracy. The following provides detailed descriptions.

That the terminal generates the first test signal based on the feedback speed and the preset first volume, and sends the first test signal to the hearing-assistive device in 302 includes:
F1: Generate, based on the feedback speed, the preset first volume, and a preset volume increase/decrease difference, a plurality of first test signals corresponding to the first frequency, and send the plurality of first test signals to the hearing-assistive device, where a volume of one first test signal in the plurality of first test signals is the first volume, and a volume of a first test signal other than the one first test signal in the plurality of first test signals is determined based on the first volume and the volume increase/decrease difference.

The terminal generates the plurality of first test signals, and the terminal may send the plurality of first test signals in sequence, and the hearing-assistive object may send the plurality of corresponding first feedback instructions for the plurality of first test signals. For example, the terminal may generate two first test signals separately, and then send the two first test signals to the hearing-assistive device in sequence. The hearing-assistive device plays one first test signal when receiving the first test signal, and the hearing-assistive object generates one first feedback instruction for the played first test signal, and feeds back, by using the first feedback instruction, whether the hearing-assistive object hears the first test signal.

Volumes of the plurality of first test signals generated by the terminal may be separately obtained based on the preset first volume and the volume increase/decrease difference. The preset first volume may be a preset initial volume, and the volume increase/decrease difference is used to adjust the first volume. The volume increase/decrease difference may be specifically a fixed volume difference, or may be a set including a plurality of volume differences. The volume of one first test signal in the plurality of first test signals is the first volume, and the volume of the first test signal other than the one first test signal in the plurality of first test signals is determined based on the first volume and the volume increase/decrease difference. For example, in this embodiment of this application, the first volume may be adjusted to obtain a second volume. For example, the second volume may be equal to a volume obtained by adding the volume increase/decrease difference to the first volume. In addition, a third volume may be obtained based on the second volume and the volume increase/decrease difference, and so on.

For example, the volume increase/decrease difference may be increase by 5 dB, decrease by 5 dB, decrease by 10 dB, or the like. The terminal device generates a 1^{st} first test signal based on the initial volume, increases the initial volume by 5 dB to obtain a 2^{nd} first test signal, decreases a volume of the 2^{nd} first test signal by 10 dB to obtain a 3^{rd} first test signal, so that fast iteration of the first test signal can be implemented, increases a volume of the 3^{rd} first test signal by 5 dB to obtain a 4^{th} first test signal, and then decreases a volume of the 4^{th} first test signal by 5 dB to obtain a 5^{th} first test signal. In this embodiment of this application, the first test signal is iteratively increased and decreased for a plurality of times, so that audiometry accuracy can be improved.

In the foregoing implementation scenario in which F1 is performed, obtaining the hearing loss test result based on the first test signal and the first feedback instruction in 303 includes:

G1: Determine a first flip point based on a plurality of first feedback instructions corresponding to the plurality of first test signals, where the first flip point includes a time point at which two adjacent first feedback instructions in the plurality of first feedback instructions indicate different listening results.

When the hearing-assistive device plays one first test signal in the plurality of first test signals, the hearing-assistive device sends one piece of first feedback signaling to the terminal, and the terminal may detect the piece of first feedback signaling. When the hearing-assistive device plays another first test signal, the hearing-assistive device sends another piece of first feedback signaling to the terminal, and the terminal may detect the another piece of first feedback signaling, and determine the first flip point based on the plurality of detected first feedback instructions, where the first flip point includes the time point at which the two adjacent first feedback instructions in the plurality of first feedback instructions indicate the different listening results, and each of the plurality of first feedback instructions may indicate a listening result of the hearing-assistive object. For example, a listening result is "hearing a sound", and another listening result different from the listening result is "hearing no sound". For example, when the hearing-assistive device plays a current first test signal, if the hearing-assistive object hears current signal playing, the hearing-assistive object taps a test button of "hearing a sound". When the hearing-assistive device plays a next first test signal, if the hearing-assistive object does not hear next signal playing, the hearing-assistive object does not tap a test button of "hearing a sound". That is, a time point from hearing to hearing failure is referred to as the first flip point. Therefore, the terminal may determine the first flip point by detecting whether listening results respectively indicated by two adjacent first feedback instructions are the same.

G2: The terminal obtains a second flip point adjacent to the first flip point.

The terminal may determine the second flip point in a manner of detecting a flip point described in G1, where the second flip point is a flip point adjacent to the first flip point. For example, the second flip point is a second flip point that is determined before the first flip point and that is adjacent to the first flip point. Whether the hearing loss test result of the first frequency is accurate may be determined by using two adjacent flip points. Specifically, the following steps G3 and G4 may be performed.

G3: When a difference between the first flip point and the second flip point is greater than a preset sound pressure threshold, clear a frequency test result corresponding to the first flip point.

G4: When a difference between the first flip point and the second flip point is less than or equal to a preset sound pressure threshold, obtain the hearing loss test result based on a frequency test result of the first flip point.

The terminal may record the difference between the first flip point and the second flip point that are adjacent to each other. If the difference is greater than the preset sound pressure threshold, it indicates that the currently recorded frequency test result of the first flip point is inaccurate, and the frequency test result corresponding to the first flip point is cleared. If the difference is less than or equal to the preset sound pressure threshold, it indicates that the currently recorded frequency test result of the first flip point is accurate, and a frequency test may continue to be performed. The frequency test result of the first flip point may be used to obtain the hearing loss test result.

It may be understood that the foregoing process of G1 to G4 may be referred to as a flip point iterative determining process. The following describes an application scenario of G1 to G4 by using an example. When the flip point is determined, volumes of the first two test signals may be transitioned from being increased by 5 dB to being decreased by 10 dB, and fast iteration is performed. Subsequently, volumes of two test signals are transitioned from being increased by 5 dB to being decreased by 5 dB. Audiometry accuracy can be improved by dynamically adjusting the volume of the test signal. In a test process, a difference between adjacent flip points is recorded for a current frequency. If the difference is greater than the sound pressure threshold, a hearing loss test result of the flip point is cleared and the test is continued. After flipping is performed for five times, an average value of the last three flip points is used as a hearing loss test result of the current frequency.

In some embodiments of this application, obtaining the hearing loss test result based on the frequency test result of the first flip point in G4 includes:
H1: When a quantity of times that the frequency test result reaches an extreme value exceeds a times threshold, and/or a quantity of first test signals sent at the first frequency exceeds a quantity threshold, determine the hearing loss test result based on the frequency test result of the first flip point.

The terminal may further set a frequency test end condition. A test on the first frequency is used as an example, the terminal obtains the frequency test result of the first flip point, and then the terminal determines whether the frequency test result meets the preset frequency test end condition, where the frequency test end condition includes that the quantity of times that the frequency test result reaches the extreme value exceeds the times threshold, and/or the quantity of first test signals sent at the first frequency exceeds the quantity threshold. When the frequency test result meets the preset frequency test end condition, the terminal stops testing the first frequency, and determines the hearing loss test result based on the frequency test result of the first flip point. The frequency is tested to improve efficiency of the hearing loss test.

For example, to improve test efficiency, when a frequency is tested and a frequency test result reaches an extreme value (for example, a maximum value and a minimum value) for three consecutive times or reaches an extreme value for a total of six times, the test on the frequency is ended and the extreme value is used as a hearing loss test result of the frequency. When a total quantity of rounds of tests on a frequency reaches 30, the test on the frequency is ended, and an average value of frequency test results of the last three flip points is used as a hearing loss test result of the frequency.

Further, in some embodiments of this application, in addition to the foregoing step of the hearing loss compensation method, the hearing loss compensation method performed by the terminal further includes the following step.

I1: When the frequency test result exceeds a frequency test average result and reaches a preset threshold, the terminal performs feedback instruction detection at the first frequency again.

The terminal may further obtain the frequency test average result. After obtaining the frequency test result corresponding to the first frequency, the terminal compares the frequency test result corresponding to the first frequency with the frequency test average result. When the frequency test result exceeds the frequency test average result and reaches the preset threshold, the terminal performs feedback instruction detection at the first frequency again. Accuracy of the hearing loss test can be improved by determining whether to perform detection again at the first frequency.

By using the foregoing example for description, the terminal may generate the hearing loss test result in a plurality of manners. For example, the terminal may obtain the accurate hearing loss test result in at least one of the following manners: a feedback speed of the hearing-assistive object, iterative determining of a flip point, and re-testing of an extreme value point. Specifically, the terminal may select at least one of the foregoing manners with reference to an application scenario to obtain the accurate hearing loss test result.

304: The terminal performs real-time compensation processing in time domain and/or frequency domain based on the hearing loss test result to obtain a first compensation gain.

After obtaining the hearing loss test result, the terminal may perform real-time compensation processing on the hearing-assistive object. Specifically, the terminal may perform real-time compensation processing in time domain and/or frequency domain to obtain the first compensation gain, and may extract a microphone input signal envelope from the first compensation gain by performing real-time compensation processing. A specific used compensation manner is not limited herein.

In some embodiments of this application, that the terminal performs real-time compensation processing in time domain and/or frequency domain based on the hearing loss test result to obtain the first compensation gain in 304 includes:
J1: The terminal obtains a first hearing loss value at the first frequency in the hearing loss test result.

The terminal may perform real-time compensation processing at each frequency. The first frequency is used as an example. The terminal obtains the first hearing loss value at the first frequency in the hearing loss test result.

J2: The terminal determines, based on a mapping relationship between a hearing loss value and a correction gain value, a first correction gain value corresponding to the first hearing loss value in time domain and/or frequency domain.

The terminal may perform real-time compensation in time domain to obtain the first correction gain value in time domain in the hearing loss test result. Alternatively, the terminal may perform real-time compensation in frequency domain to obtain the first correction gain value in frequency domain in the hearing loss test result. Alternatively, the terminal may separately perform real-time compensation in time domain and frequency domain to separately obtain the first correction gain value in time domain and the first correction gain value in frequency domain.

The mapping relationship between the hearing loss value and the correction gain value refers to a polynomial formula between the hearing loss value and the correction gain value. The polynomial formula between the hearing loss value and the correction gain value may also be referred to as a fitting formula between the hearing loss value and the correction gain value, which is referred to as a fitting formula briefly in the following. The terminal may determine, based on the mapping relationship between the hearing loss value and the correction gain value, the first correction gain value corresponding to the first hearing loss value in time domain and/or frequency domain. In this embodiment of this application, whether the terminal performs real-time compensation in time domain or performs real-time compensation in frequency domain or performs real-time compensation in both time domain and frequency domain is determined with reference to an application scenario.

J3: The terminal determines the first compensation gain based on the first compensation gain value.

After obtaining the first compensation gain value, the terminal determines the first compensation gain based on the first compensation gain value. For example, the terminal may obtain a plurality of groups of first compensation gain values by performing J1 and J2 for a plurality of times, and the plurality of groups of first compensation gain values may form the first compensation gain. In this embodiment of this application, real-time compensation may be performed based on the mapping relationship between the hearing loss value and the correction gain value, to improve accuracy of hearing loss compensation.

Further, in some embodiments of this application, that the terminal performs real-time compensation processing in time domain and/or frequency domain based on the hearing loss test result to obtain the first compensation gain in 304 further includes:
J4: The terminal determines a hearing curve based on the hearing loss test result.

J5: The terminal determines the mapping relationship between the hearing loss value and the correction gain value based on a classification result corresponding to the hearing curve.

The hearing loss test result may include one or more hearing curves. The terminal may obtain the hearing curve, classify a type of the hearing curve based on a predetermined hearing curve library, to obtain the classification result corresponding to the hearing curve, and select, based on the classification result, the corresponding mapping relationship between the hearing loss value and the correction gain value, so that the mapping relationship between the hearing loss value and the correction gain value can be used for real-time compensation.

For example, when the hearing curve is of a linear attenuation type, a high-frequency decrease type, or a basin-shaped type, different coefficients may be set for the mapping relationship between the hearing loss value and the compensation gain value to match the hearing curve corresponding to the hearing-assistive object. Therefore, the corresponding mapping relationship between the hearing loss value and the correction gain value may be queried based on the hearing curve in the hearing loss test result.

Further, in some embodiments of this application, that the terminal determines, based on the mapping relationship between the hearing loss value and the correction gain value, the first correction gain value corresponding to the first hearing loss value in time domain and/or frequency domain in J2 includes:
J21: The terminal divides, based on an octave, an external input signal collected by the hearing-assistive device into a plurality of subbands.

The external input signal collected by the hearing-assistive device by using a microphone is a time-domain signal, is converted into a frequency-domain signal through Fourier transform, and then selects data in different frequency bands based on the octave (that is, a frequency domain multiplication relationship), to obtain the plurality of subbands. For example, the frequency domain multiplication relationship may specifically include a frequency domain such as 125 Hz, 250 Hz, 500 Hz, 1000 Hz, 2000 Hz, 4000 Hz and 8000 Hz.

In some embodiments of this application, that the terminal determines, based on the mapping relationship between the hearing loss value and the correction gain value, the first correction gain value corresponding to the first hearing loss value in J2 further includes: The terminal performs human voice separation on the external input signal collected by the hearing-assistive device, to obtain a human voice signal; and performs wide dynamic range compression compensation and envelope enhancement on the human voice signal to obtain a compensated and enhanced human voice signal.

After obtaining the external input signal, the terminal may further perform hearing-assistive enhancement, perform artificial intelligence (artificial intelligence, AI) separation on the external input signal, and perform wide dynamic range compression compensation and envelope enhancement on the extracted human voice signal. Therefore, a better human voice compensation effect can be provided, and a speech recognition rate can be improved.

Specifically, that the terminal divides, based on the octave, the external input signal collected by the hearing-assistive device into the plurality of subbands in J21 includes: The terminal divides the compensated and enhanced human voice signal into a plurality of subbands based on the octave.

Wide dynamic range compression compensation and envelope enhancement are performed on the human voice signal to obtain the compensated and enhanced human voice signal. The human voice signal may be used for subsequent correction gain calculation, so that the better human voice compensation effect can be provided, and the speech recognition rate can be improved.

J22: The terminal obtains speech estimation signals and noise estimation signals corresponding to the plurality of subbands.

The terminal may estimate speech signals and noise signals of different subbands to obtain the speech estimation signals and the noise estimation signals corresponding to the plurality of subbands.

J23: The terminal performs speech intelligibility index (speech intelligibility index, SII) analysis based on the speech estimation signals and the noise estimation signals to obtain loudness of hearing loss.

The terminal transmits the speech estimation signals and the noise estimation signals to an SII analysis module included in the terminal to perform intelligibility optimization analysis, to obtain loudness of the speech signals and the noise signals, where the loudness of the speech signals and the noise signals is also referred to as the loudness of the hearing loss.

J24: The terminal determines a first frequency domain compensation gain of the first hearing loss value in frequency domain based on the mapping relationship between the hearing loss value and the correction gain value.

The terminal may perform real-time compensation in frequency domain to obtain the first hearing loss value in frequency domain in the hearing loss test result, and then determine the first frequency domain compensation gain of the first hearing loss value in frequency domain based on the mapping relationship between the hearing loss value and the correction gain value.

J25: The terminal performs iterative optimization on the first frequency domain compensation gain based on the speech estimation signals and the noise estimation signals corresponding to the plurality of subbands and the loudness of the hearing loss, to obtain a second frequency domain compensation gain that maximizes intelligibility within preset loudness, where the second frequency domain compensation gain belongs to the first correction gain value.

The terminal may obtain energy of the speech estimation signals corresponding to the plurality of subbands and energy of the noise estimation signals corresponding to the plurality of subbands, perform iterative optimization on the first frequency domain compensation gain based on the energy of the speech estimation signals and the noise estimation signals of different subbands and the loudness of the hearing loss, to obtain the second frequency domain compensation gain that maximizes intelligibility within the preset loudness, and determine the first correction gain value based on the second frequency domain compensation gain. In this embodiment of this application, accuracy of hearing loss compensation can be improved by using the foregoing hearing loss compensation in frequency domain.

Further, in some embodiments of this application, that the terminal determines, based on the mapping relationship between the hearing loss value and the correction gain value, the first correction gain value corresponding to the first hearing loss value in time domain and/or frequency domain in J2 includes:
J26: The terminal determines a first time domain compensation gain of the first hearing loss value in time domain based on the mapping relationship between the hearing loss value and the correction gain value.

The terminal may perform real-time compensation in time domain to obtain the first hearing loss value in the hearing loss test result, and then determine the first time domain compensation gain of the first hearing loss value in time domain based on the mapping relationship between the hearing loss value and the correction gain value.

J27: The terminal extracts envelope information of an external input signal collected by the hearing-assistive device.

The hearing-assistive device obtains the envelope information of the external input signal, where the external input signal collected by using the microphone is a time-domain signal.

J28: When an average value of the envelope information is greater than a preset envelope threshold, the terminal performs linear attenuation processing on the first time domain compensation gain to obtain a second time domain compensation gain, where the second time domain compensation gain belongs to the first correction gain value.

A value of the envelope threshold is not limited, and the average value of the envelope information is greater than the envelope threshold. The terminal performs linear attenuation processing on the first time domain compensation gain, obtains the second time domain compensation gain through the linear attenuation processing, and determines the first correction gain value based on the second time domain compensation gain, so that linear attenuation occurs on an envelope of the external input signal, to improve accuracy of hearing loss compensation.

J29: When an average value of the envelope information is less than or equal to a preset envelope threshold, the terminal performs linear improvement processing on the first time domain compensation gain to obtain a third time domain compensation gain, where the third time domain compensation gain belongs to the first correction gain value.

The average value of the envelope information is less than or equal to the envelope threshold. The terminal performs linear improvement processing on the first time domain compensation gain, obtains the third time domain compensation gain through the linear improvement processing, and determines the first correction gain value based on the third time domain compensation gain, so that linear improvement occurs on an envelope of the external input signal, to improve accuracy of hearing loss compensation.

There is no sequence relationship between J21 to J25 and J26 to J29.

By using the foregoing example for description, the terminal may generate the first correction gain value in a plurality of manners. For example, the terminal may obtain the accurate first compensation gain value in at least one of the following manners: obtaining the loudness of the hearing loss through intelligibility analysis, and then performing frequency domain compensation based on the loudness of the hearing loss; and performing real-time envelope time domain enhancement. Specifically, the terminal may select at least one of the foregoing manners with reference to an application scenario to obtain the accurate first correction gain value, so that a fitting effect can be better improved and the speech recognition rate can be enhanced.

305: The terminal performs speech correction on the first compensation gain based on a preset speech test signal to obtain the hearing loss compensation gain.

After determining the first compensation gain, the terminal may send the preset speech test signal to the hearing-assistive device, and perform speech correction on the first compensation gain based on the speech test signal to obtain the hearing loss compensation gain. The speech correction can improve the speech recognition rate.

In some embodiments of this application, that the terminal performs speech correction on the first compensation gain based on the preset speech test signal to obtain the hearing loss compensation gain in 305 includes:
K1: The terminal generates a speech test signal based on an audio spectral centroid in a frequency band division manner, where the speech test signal includes test words at a plurality of frequencies corresponding to different audio spectral centroids.

K2: The terminal sends the speech test signal to the hearing-assistive device.

K3: The terminal performs speech correction on the first compensation gain based on the speech test signal and a third feedback instruction to obtain the hearing loss compensation gain, where the third feedback instruction includes a feedback instruction detected by the terminal when the hearing-assistive device plays the third test signal.

The terminal generates the speech test signal based on the audio spectral centroid in the frequency band division manner. For example, based on an initial-consonant spectral centroid in the frequency band division manner, for each type of audio spectral centroid, a test word is selected from test words at a plurality of frequencies to form the speech test signal. Subsequently, a manner of selecting the test word is referred to as "one from multiple". One from multiple means that during speech correction, a plurality of confusable words appear in a UI of the terminal, for example, four confusable words appear, and then an audio of one of the words is played, the user taps to select the heard word.

After the hearing-assistive device receives the speech test signal, the hearing-assistive device plays the speech test signal to the hearing-assistive object. If the hearing-assistive object hears audio content of the speech test signal, the hearing-assistive object may send the third feedback instruction to the terminal. For example, the hearing-assistive object taps a feedback button in the UI, so that the terminal detects the third feedback instruction. The terminal performs speech correction on the first compensation gain based on the speech test signal and the third feedback instruction to obtain the hearing loss compensation gain. The speech test signal is generated in the foregoing manner of one from multiple, and the speech test signal is used for speech correction, so that accuracy of hearing loss compensation can be improved.

In some embodiments of this application, that the terminal performs speech correction on the first compensation gain based on the preset speech test signal in 305 includes:
K4: The terminal obtains the speech test signal from a preset corpus, where the corpus includes a plurality of groups of speech test signals, each group of speech test signals includes initial consonant-based single-character groups divided into a same group based on an audio spectral centroid and different octaves, and one initial consonant-based single-character group includes two or more randomly extracted confusable words.

The terminal obtains the corpus. The corpus provided in this embodiment of this application may include the plurality of groups of speech test signals, each group of speech test signals includes initial consonant-based single-character groups divided into the same group based on the audio spectral centroid and the different octaves, and one initial consonant-based single-character group includes two or more randomly extracted confusable words. A quantity of confusable words included in one initial consonant-based single-character group is not limited.

K5: The terminal plays the two or more confusable words in the same initial consonant-based single-character group in a random order through the hearing-assistive device.

The terminal controls the hearing-assistive device to play the two or more confusable words in the same initial consonant-based single-character group in the random order in the speech test signal, and the hearing-assistive object may send a feedback instruction to the terminal when hearing the confusable words randomly played by the hearing-assistive device.

For example, the terminal may obtain the corpus in advance. The corpus is divided into 15 groups of speech test signals based on an initial consonant-based single-character, a spectral centroid, and an octave. The 15 groups are randomly played. Four test words are randomly extracted from each group, and the four test words are played in the random order, to resolve a problem of speech material reuse effectiveness. In addition, five groups of feedbacks are set, and differentiated requirements of different users for volumes, timbre equalization, noise reduction, and the like in a feedback form of one from two.

K6: The terminal detects a fourth feedback instruction sent by the hearing-assistive object.

K7: The terminal obtains a consistency error frequency band of the hearing-assistive object based on the fourth feedback instruction and the speech test signal.

When detecting the fourth feedback instruction, the terminal obtains the consistency error frequency band based on the speech test signal and the fourth feedback instruction, where the consistency error frequency band is a frequency band in which a consistency error occurs and that is determined by the terminal based on the speech recognition rate.

K8: The terminal adjusts the first compensation gain based on the consistency error frequency band.

The terminal adjusts the first compensation gain based on the consistency error frequency band to obtain the hearing loss compensation gain. Each group of words is randomly extracted and played in the random order, so that the problem of speech material reuse effectiveness can be resolved. The consistency error frequency band can be determined based on the fourth feedback instruction, to meet differentiated requirements of different users for volumes, timbre equalization, noise reduction, and the like. The speech test signal is generated, and the speech test signal is used for speech correction, so that accuracy of hearing loss compensation can be improved.

There is no sequence relationship between K1 to K3 and K4 to K8.

306: The terminal device sends the hearing loss compensation gain to the hearing-assistive device, where the hearing-assistive device is configured to perform hearing loss compensation on a first speech signal based on the hearing loss compensation gain to obtain a second speech signal.

After the terminal obtains the hearing loss compensation gain, the terminal sends the hearing loss compensation gain through the communication connection to the hearing-assistive device, and the hearing-assistive device receives and stores the hearing loss compensation gain.

Specifically, that the terminal device sends the hearing loss compensation gain to the hearing-assistive device in 306 includes:
The terminal performs hearing loss compensation on the to-be-transmitted first speech signal based on the hearing loss compensation gain to obtain the second speech signal, and sends the second speech signal to the hearing-assistive device.

The terminal may perform hearing loss compensation on the first speech signal, and then the hearing-assistive device sends the second speech signal obtained after the hearing loss compensation, so that the hearing-assistive device receives the second speech signal obtained after the hearing loss compensation, and the hearing-assistive device needs to play only the second speech signal, so that a processing procedure of the hearing-assistive device can be simplified.

In some embodiments of this application, in addition to the foregoing steps of the hearing loss compensation method, the hearing loss compensation method performed by the terminal further includes the following steps.

L1: The terminal detects a fifth feedback instruction sent by the hearing-assistive object when the hearing-assistive device plays the second speech signal.

L2: The terminal determines a listening parameter of the hearing-assistive object based on the fifth feedback instruction, where the listening parameter includes at least one of the following: a listening volume, a noise reduction degree, and a frequency band-specific gain.

After the hearing-assistive device plays the second speech signal, if the hearing-assistive object hears audio content of the second speech signal, the hearing-assistive object may send the fifth feedback instruction to the terminal. For example, the hearing-assistive object taps a feedback button in the UI of the terminal, so that the terminal detects the fifth feedback instruction. The terminal determines the listening parameter of the hearing-assistive object based on the fifth feedback instruction, where the hearing parameter includes the listening volume, and further include the noise reduction degree, the frequency band-specific gain, and the like. The terminal may determine the listening parameter of the hearing-assistive object by detecting the fourth feedback instruction, where the listening parameter may be used to describe an actual auditory sense of the hearing-assistive object, so that accuracy of hearing loss compensation can be tested.

Further, in some embodiments of this application, in addition to the foregoing steps of the hearing loss compensation method, the hearing loss compensation method performed by the terminal further includes the following step.

M1: The terminal performs pure tone audiometry (pure tone audiometry, PTA) based on the hearing loss compensation gain and the listening parameter of the hearing-assistive object to obtain an audiometry report of the hearing-assistive object.

The terminal performs PTA processing on the hearing-assistive object based on the hearing loss compensation gain and the listening parameter of the hearing-assistive object, and outputs a fitting report. The fitting report includes a hearing loss curve chart of the hearing-assistive object, classification of hearing loss levels, a speech recognition rate before gain compensation, and a speech recognition rate after gain compensation.

By using the foregoing example for description, the terminal may generate the hearing loss compensation gain in a plurality of manners. For example, the terminal may obtain the accurate hearing loss compensation gain in at least one of the following manners: time-frequency domain real-time compensation, speech correction, and feedback adjustment. Specifically, the terminal may select at least one of the foregoing manners with reference to an application scenario to obtain the accurate hearing loss compensation gain, so that the fitting effect can be better improved, and the speech recognition rate can be enhanced.

It can be learned from the descriptions of the hearing loss compensation method performed by the terminal in the foregoing embodiment that the terminal may perform hearing loss audiometry, fitting formula compensation, a speech correction algorithm, and feedback adjustment. Hearing loss audiometry is set on a terminal side to obtain the hearing loss test result of the user. Fitting formula compensation is set on a headset side, and real-time compensation is performed based on the hearing loss test result and an external signal. Speech correction and feedback adjustment are set on a mobile phone side, and the hearing loss compensation gain is adjusted in a personalized manner based on the speech recognition rate and a feedback of the hearing-assistive object. After fitting is completed, the terminal may output a hearing report, where the hearing report includes information such as a hearing loss curve of the hearing-assistive object and a fitted speech recognition rate. According to the foregoing procedure, an embodiment of this application provides an autonomous fitting solution. A user does not need to go to a hearing center to search for a professional hearing aid dispenser for fitting, and can independently complete an entire fitting procedure, and achieve a good hearing-assistive effect.

For better understanding and implementation of the foregoing solutions in embodiments of this application, specific descriptions are provided below by using corresponding application scenarios as examples.

Embodiments of this application provide a hearing loss compensation system. The hearing loss compensation system may include a terminal and a hearing-assistive device. Some possible implementations of this application in embodiments of this application exist between a mobile phone and a Bluetooth headset, between the mobile phone and a wired headset, and between the mobile phone and a hearing aid. This application scenario may also exist between a tablet/notebook computer/desktop computer and a Bluetooth headset/wired headset/hearing aid. Subsequently, an example in which the terminal is specifically a mobile phone and the hearing-assistive device is specifically a headset is used, and a hearing-assistive object is specifically a user who operates the mobile phone and wears the headset.

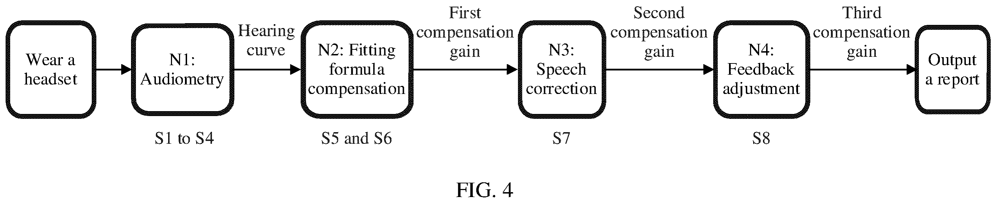

A system architecture in embodiments of this application is shown in FIG. 4, and mainly includes the following procedures: wearing a headset, N1: hearing loss audiometry, N2: fitting formula compensation, N3: speech correction, N4: feedback adjustment and output of a fitting report.

A main method procedure includes S1 to S8 shown in the following. An N1 procedure includes S1 to S4, an N2 procedure includes S5 and S6, an N3 procedure includes S7, and an N4 procedure includes S8. The following briefly describes the following steps S1 to S8.

S1: A user wears a headset, and first detects a feedforward (FeedForward, FF)/feedback (FeedBack, FB) signal based on a prompt tone, and detects wearing consistency. If wearing is good, perform S2; otherwise, play a prompt tone 1 to the user.

S2: After the wearing consistency is detected, detect an ambient background noise. If the ambient background noise is less than a noise threshold, perform S3; otherwise, play a prompt tone 2 to the user.

S3: Play a pure-tone test signal to the user, detect a tap speed of the user, and use the tap speed for subsequent hearing loss compensation, where the tap speed may also be referred to as a feedback speed.

S4: Generate a test signal based on the tap speed of the user and an initial volume, and play the test signal to the user. When hearing the test signal, the user taps a feedback button in a UI of the terminal. After the test is completed, generate an audiometry result, where the audiometry result includes hearing curves of a left ear and a right ear.

S5: Classify the hearing curves based on the audiometry result, and generate a first compensation gain based on a classification result and a corresponding fitting formula, where the first compensation gain may include a frequency domain compensation gain and a time domain compensation gain.

S6: Deliver the frequency domain compensation gain and the time domain compensation gain to a WDRC module and an envelope modulation module that are included in a DSP of the headset, where the WDRC module and the envelope modulation module may be configured to amplify an uplink audio signal and a downlink audio signal based on the frequency domain compensation gain and the time domain compensation gain.

S7: Play a preset speech test signal to the user, detect a test result fed back by the user, and perform speech correction on the first compensation gain based on the test result to obtain a second compensation gain.

S8: Play a specific sentence to the user and perform feedback adjustment based on a feedback of the user, where feedback adjustment may also be referred to as a feedback test, to obtain a hearing loss compensation gain, and determine a condition such as an optimal listening volume of the user.

Finally, output a fitting report of the user.

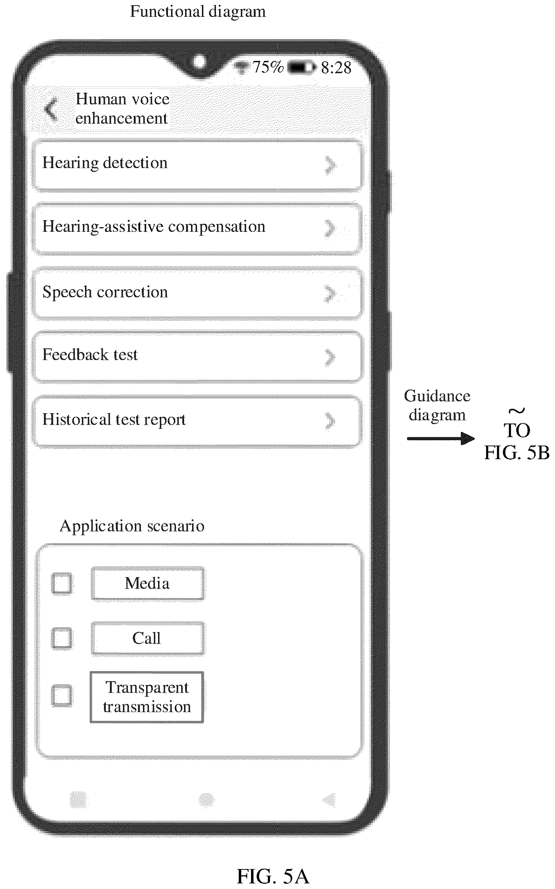

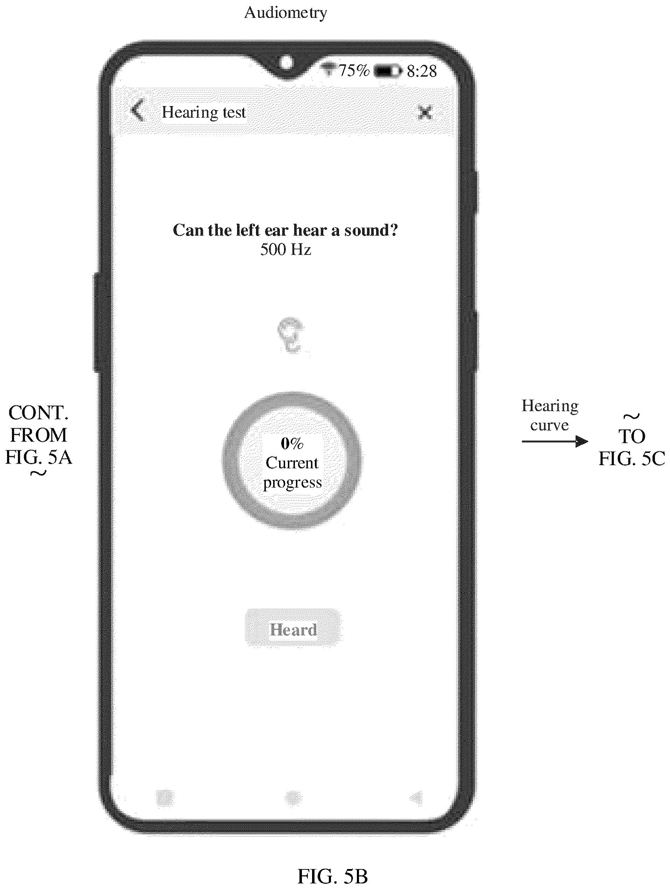

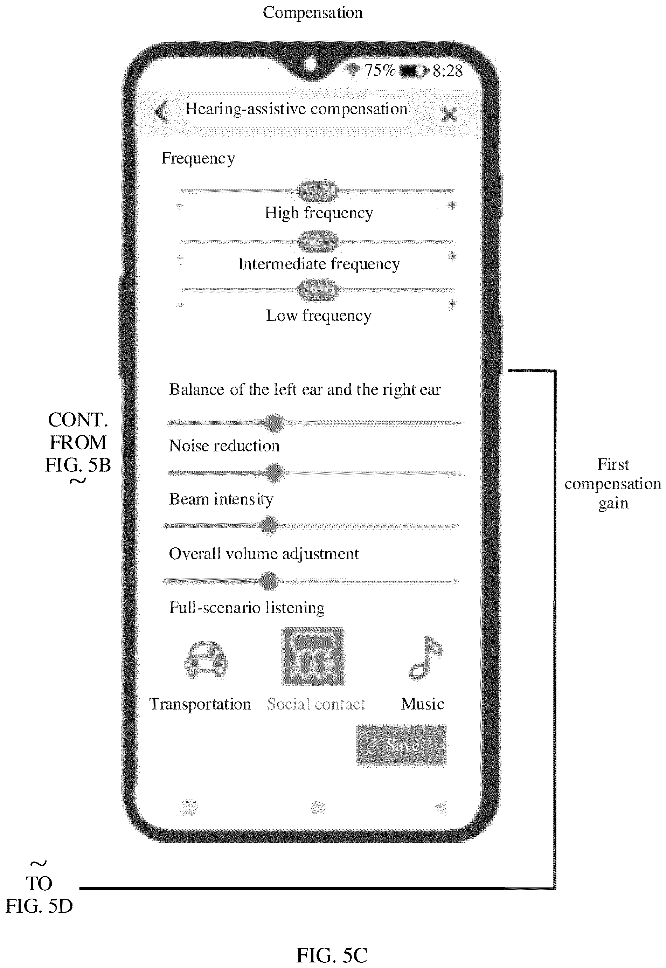

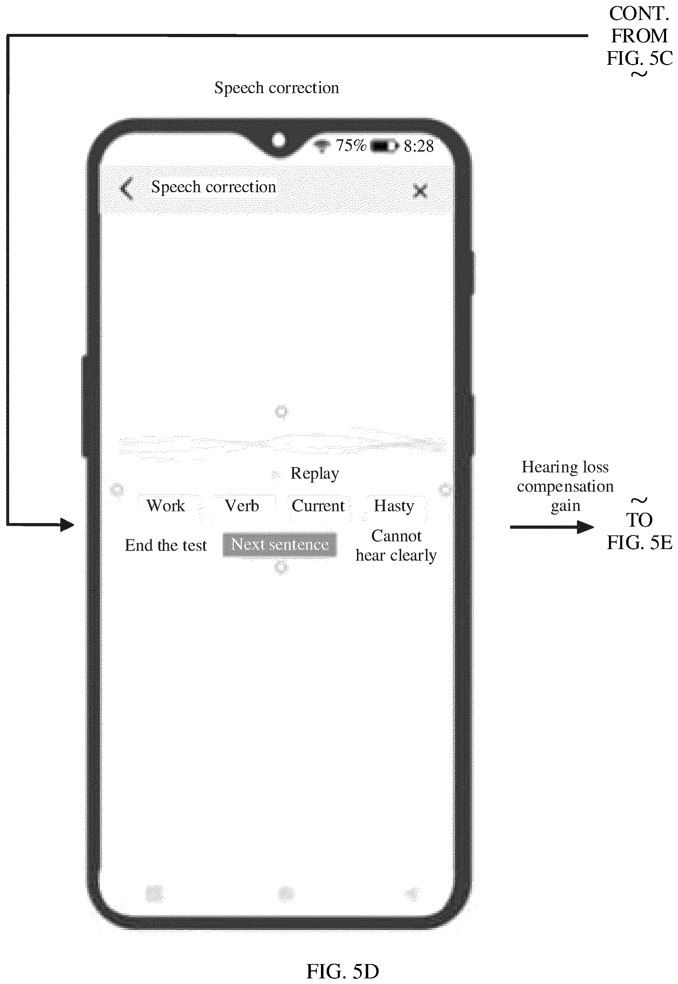

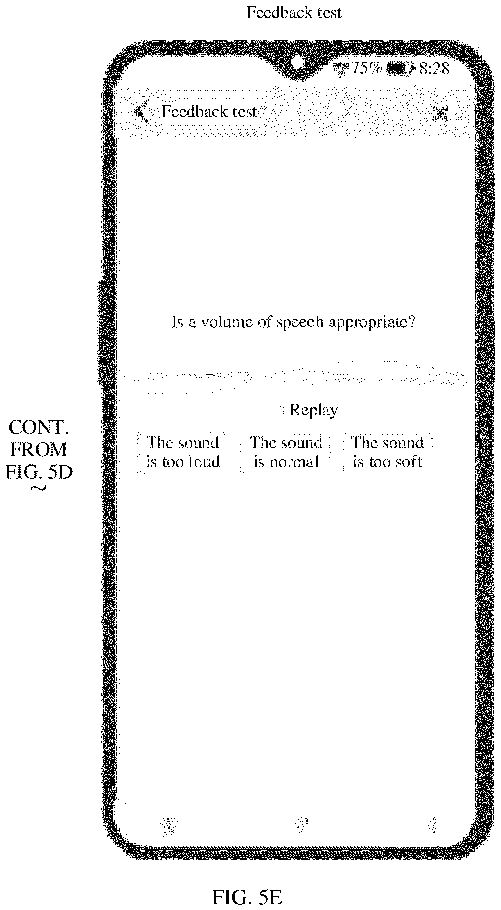

FIG. 5A to FIG. 5E are a UI function diagram according to an embodiment of this application. The function diagram may include modules such as hearing detection, hearing-assistive compensation, speech correction, a feedback test, and a historical test report. A user may select to enter a corresponding module as required. In addition, the function diagram further provides selection of an application scenario, and the user may select a scenario such as media, call, and transparent transmission as required. For example, the user may select modules such as audiometry, compensation, speech correction, and a feedback test and an application mode in the interface, and tap different modules to enter corresponding test procedures. In the audiometry module, a system plays a signal. After hearing a sound, the user taps a button of "heard". In the compensation module, compensation is performed based on a hearing curve obtained in the audiometry module to obtain a first compensation gain. Specifically, compensation may be performed on a high frequency, an intermediate frequency, and a low frequency. Balance of the left ear and the right ear, noise reduction, beam intensity, overall volume adjustment, and the like are adjusted by using a roller. In addition, the user may further select transportation, social contact, music, or other scenario in full-scenario listening, and the user may select a gain, noise reduction, a beam, different environment, and the like in different frequency band, to obtain a better gain compensation effect. In the speech correction module, the user taps a heard word, for example, four candidate words may be provided for the user to select. The speech correction module automatically adjusts a gain based on a speech recognition rate of the user, and may provide that the sound is too loud, the sound is normal, and the sound is too soft for the user to select. In the feedback test module, personalized gain adjustment is performed based on user selection.

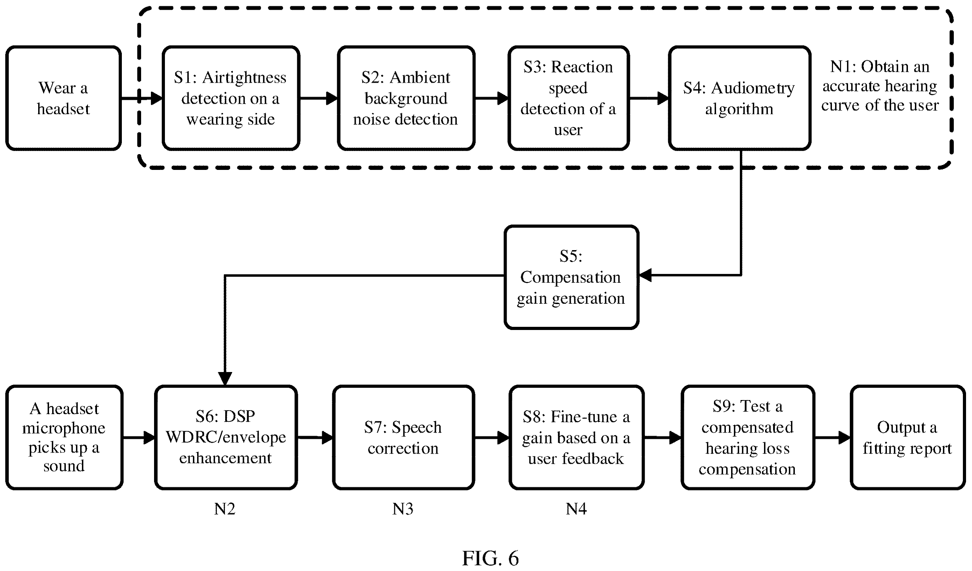

FIG. 6 provides an overall fitting procedure. A user may operate a terminal to perform autonomous fitting. Specific steps are described as follows:
N1: Obtain an accurate hearing curve of the user, which may specifically include S1 to S4.

S1: The user wears a headset, and first detects, based on a prompt tone, a sound signal collected by a microphone, for example, may be an FF/FB signal, and detects wearing consistency. If wearing is good, perform S2; otherwise, play a prompt tone 1 to the user.

S2: After the wearing consistency is detected, detect an ambient background noise. If the background noise is less than a noise threshold, perform S3; otherwise, play a prompt tone 2 to the user.

S3: Play a pure-tone test signal to the user, detect a reaction speed of the user, and input a tap speed into an audiometry algorithm in S4.

S4: Generate an audiometry test signal based on the reaction speed of the user and an initial volume, and play the audiometry test signal to the user. The user taps a feedback button when hearing the test signal. After the test is completed, generate an audiometry result, where the audiometry result includes audiometry curves of a left ear and a right ear.

S5: Classify the hearing curves based on the audiometry result, and generate a corresponding compensation gain based on a classification result and a corresponding fitting formula, where the compensation gain includes a frequency domain compensation gain and a time domain compensation gain.

S6: Deliver the frequency domain compensation gain and the time domain compensation gain to a WDRC module and an envelope modulation module that are included in a DSP of the headset, and amplify an uplink audio signal and a downlink audio signal.

S7: Play a preset speech test signal to the user in a manner of one from multiple, where an initial consonant of the speech test signal is generated based on a spectral centroid in a frequency band division manner, and fine-tune a compensation gain based on a test result. One from multiple means that during speech correction, four confusable words appear on a screen of the terminal, then an audio of one of the words is played, and the user taps to select a heard word.

S8: Play a specific sentence to the user, fine-tune a gain based on a feedback of the user, and determine a condition such as an optimal listening volume of the user.

S9: Finally, perform a PTA frequency audiometry on the user, and output a fitting report. The fitting report includes a hearing loss curve chart of the user, classification of hearing loss levels, a speech recognition rate before gain compensation, and a speech recognition rate after gain compensation.

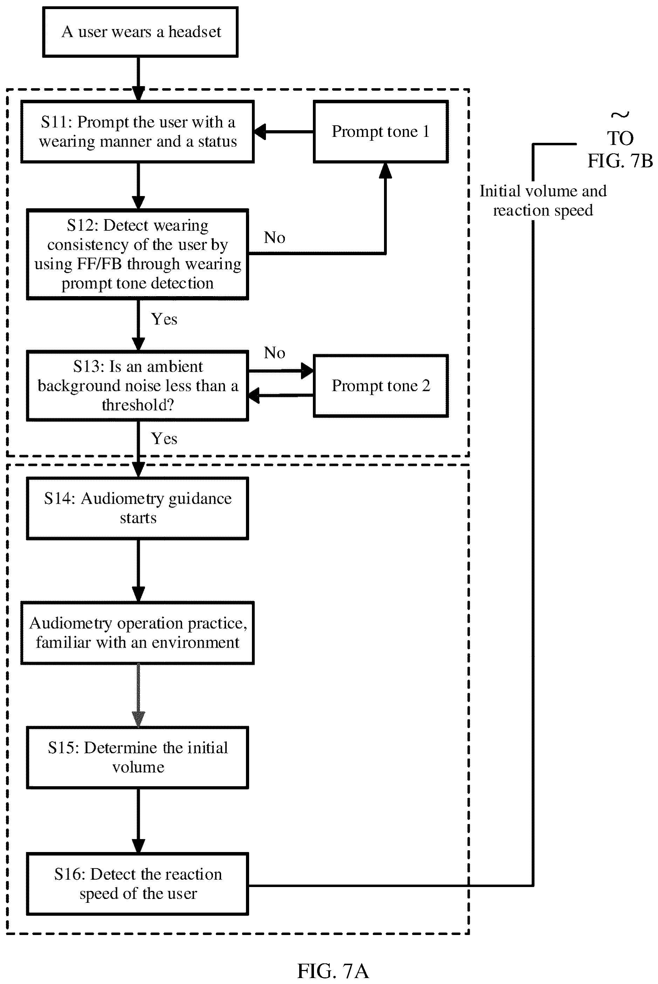

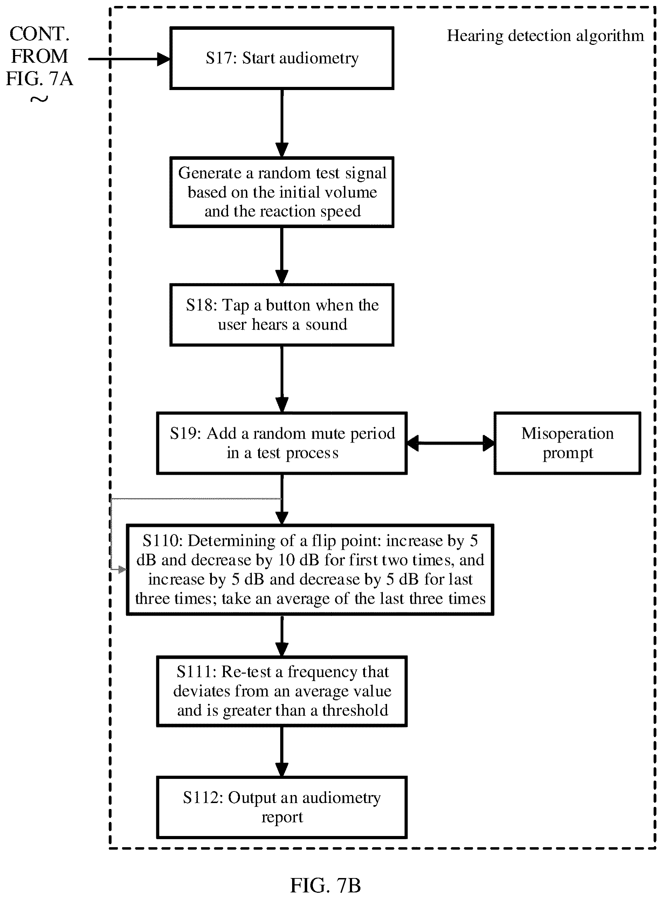

As shown in FIG. 7A and FIG. 7B, the following describes an audiometry procedure mentioned in S1 to S4. The detailed procedure includes the following steps.

S11: A user wears a headset, and prompts the user to check a wearing manner and a status.

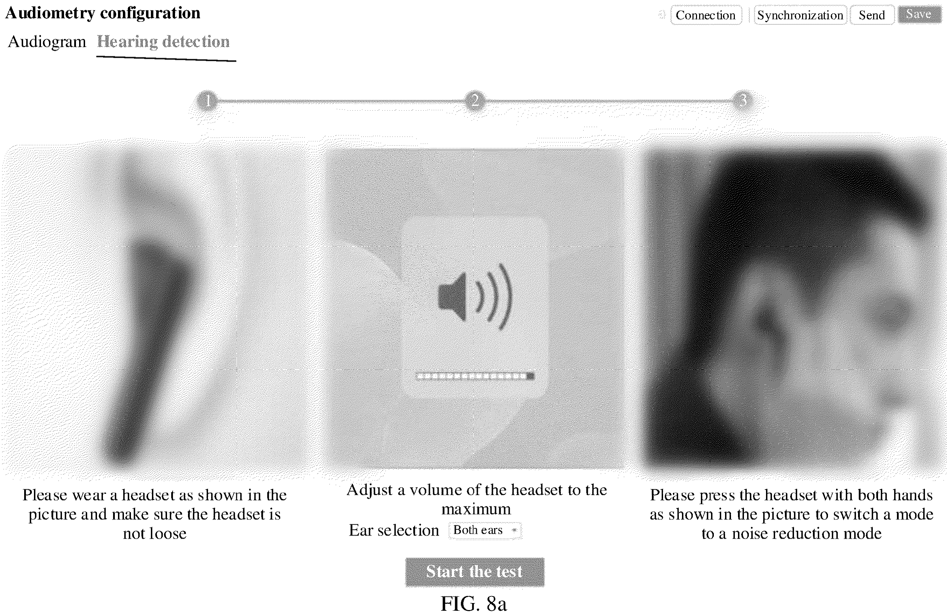

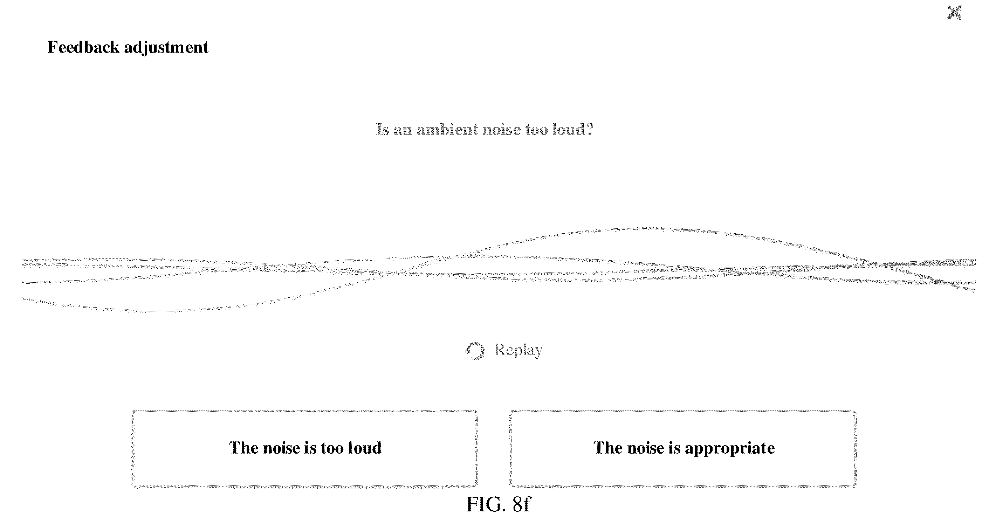

As shown in FIG. 8a, the user is first guided to perform audiometry configuration. The user can wear a headset and ensure that the headset is not loose.

S12: Detect, by using an FF/FB signal collected by a microphone (MIC), whether wearing of the wearing user is good. If the wearing is not good, prompt, by using a prompt tone 1, the user to wear again.

S13: Detect whether an ambient background noise is less than a threshold. If the ambient background noise is greater than the threshold, send a prompt tone 2 to prompt the user to search for a good environment and then perform hearing detection.

S14: Start audiometry guidance, play an audiometry practice signal, and if the signal is heard, tap a button to make the user familiar with an audiometry operation.

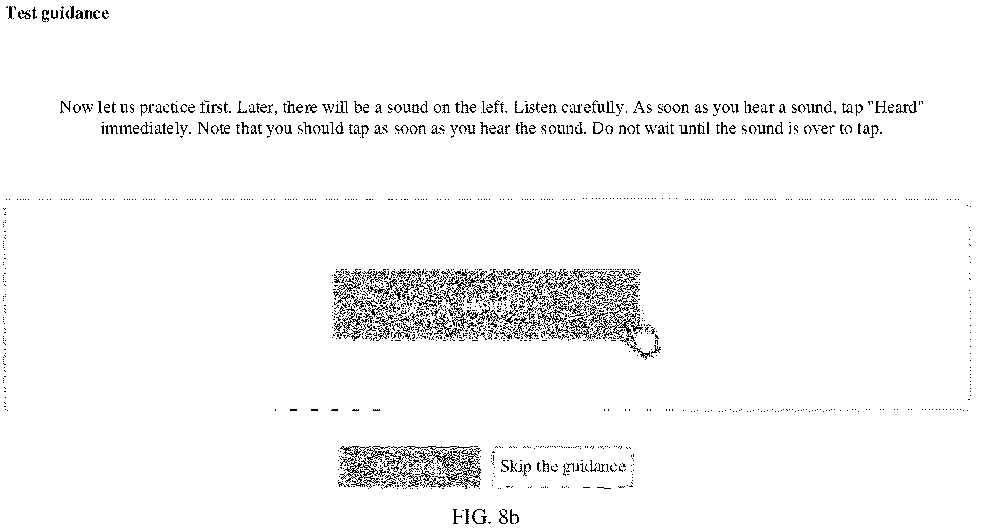

As shown in FIG. 8b, test guidance is performed on the user.

S15: Play a signal. The user adjusts, based on an auditory sense, a volume value that can be clearly heard, and uses the volume value as an initial volume for audiometry.

S16: Play a tap speed test audio, and record time from starting to play a signal to taping "heard" by the user, to obtain an average reaction speed of the user.

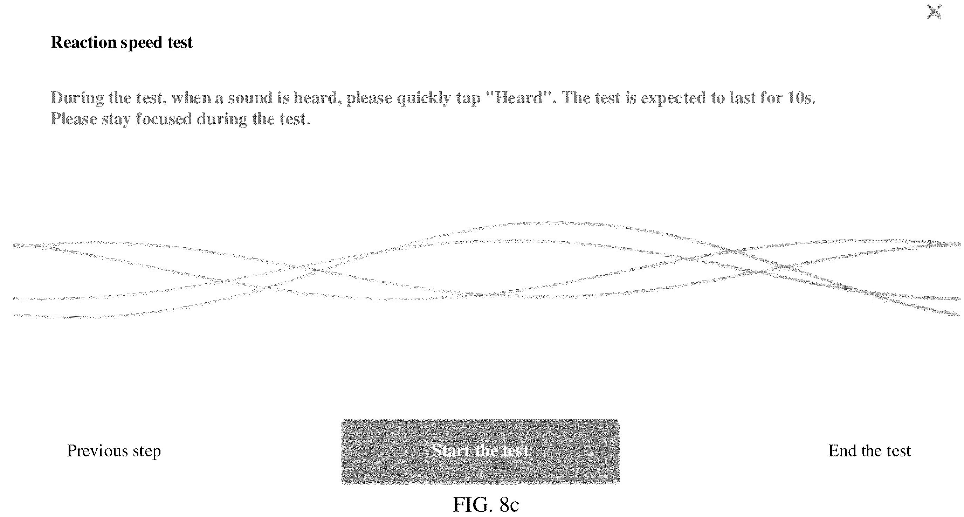

As shown in FIG. 8c, a reaction speed of the user is tested.

S17: Start audiometry, and generate a test signal with a random length within a specific range based on the initial volume and the average reaction speed, to avoid inertia fatigue of the user. The user may select monaural or binaural audiometry, and test six frequencies in sequence: 1000 Hz, 2000 Hz, 4000 Hz, 8000 Hz, 500 Hz, and 250 Hz, where 500 Hz, 1000 Hz, 2000 Hz, and 4000 Hz are key pure tone audiometry (Pure Tone Audiometry, PTA) frequencies for measuring a hearing loss level of the user.

S18: In an audiometry process, when the user hears a sound, the user taps a button, and the button is in a single state, and a test result is not displayed, to avoid a misoperation of the user and a psychological hint.

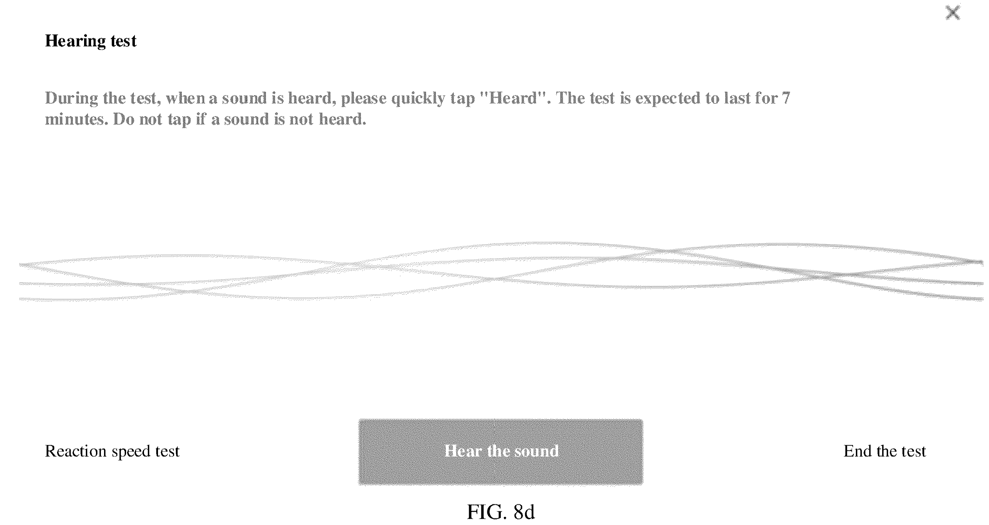

As shown in FIG. 8d, a hearing test is performed on the user. During the test, when a sound is heard, please quickly tap "heard". The test is expected to last for 7 minutes. Do not tap if a sound is not heard.

S19: A random mute period is added in a test process. When the user continuously performs a misoperation and taps a button in the mute period, play a prompt tone to the user, and the user taps the button after hearing the prompt tone.

S110:
Determining of a flip point: Volumes of the first two test signals are increased by 5 dB and then decreased by 10 dB, to perform fast iteration. Volumes of subsequent test signals are increased by 5 dB and then decreased by 5 dB to improve audiometry accuracy. In the test process, a difference between adjacent flip points is recorded. If the difference is greater than a threshold, a flip point record is cleared and the test is continued. After flipping is performed for five times, an average value of frequency test results of the last three flip points is used as a hearing loss test result of the frequency.

Frequency test end condition: to improve test efficiency, when a frequency is tested and the user reaches an extreme value (a maximum value and a minimum value) for three consecutive times or reaches an extreme value for a total of six times, the test on the frequency is ended and the extreme value is used as a hearing loss test result of the frequency. When a total quantity of rounds of tests on a frequency reaches 30, the test on the frequency is ended, and an average value of frequency test results of the last three flip points is used as a hearing loss test result of the frequency.

S111: After all frequencies are tested, calculate an average value of results of all frequencies, and re-test a frequency that deviates from the average value and is greater than the threshold.

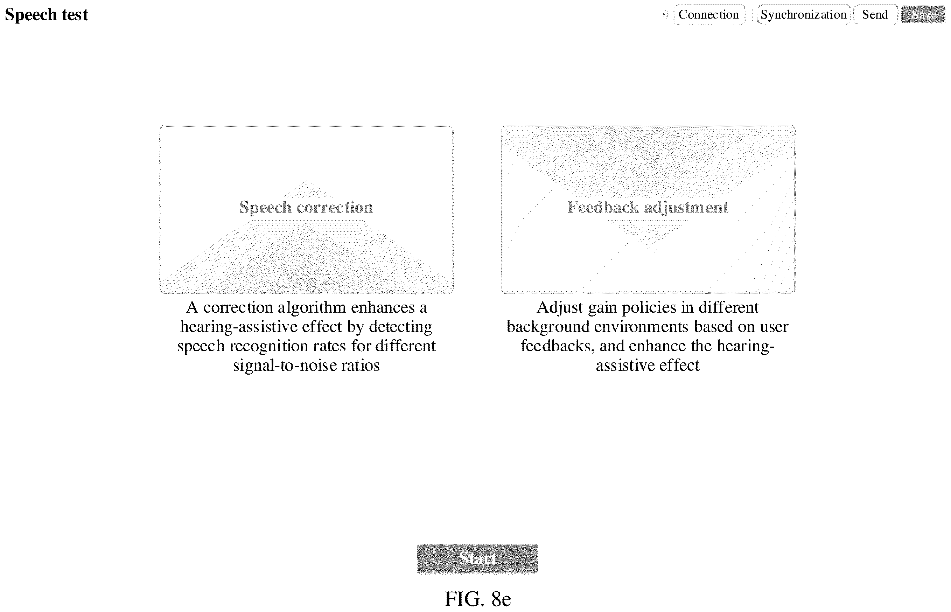

As shown in FIG. 8e, speech correction is performed on the user, and a correction algorithm enhances a hearing-assistive effect by detecting speech recognition rates for different signal-to-noise ratios. Feedback adjustment is performed on the user and gain policies in different background environments are adjusted based on user feedbacks to enhance the hearing-assistive effect.

S112: Output a test report based on the test result.

In the foregoing audiometry procedure, the tap speed of the user is detected, and the random test signal is generated based on the tap speed, to avoid inertia fatigue of the user; a single-state button test manner avoids the misoperation of the user and the psychological hint; the random mute period is added in the test process, to detect tap accuracy of the user; and iterative determining of the flip point and re-testing of the extreme value point reduce a random error and improve accuracy.

The guide procedure helps the user get familiar with the test. The test signal with the random length and the mute period are used to avoid inertia fatigue and the misoperation of the user. A flip point clearing mechanism, a frequency re-testing mechanism, and a frequency end condition are used to improve audiometry accuracy and audiometry efficiency.

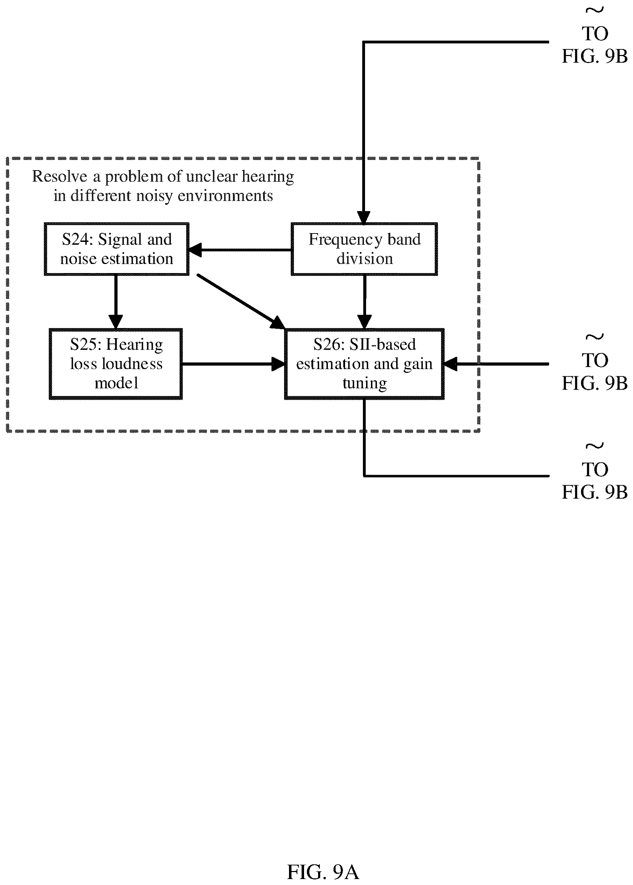

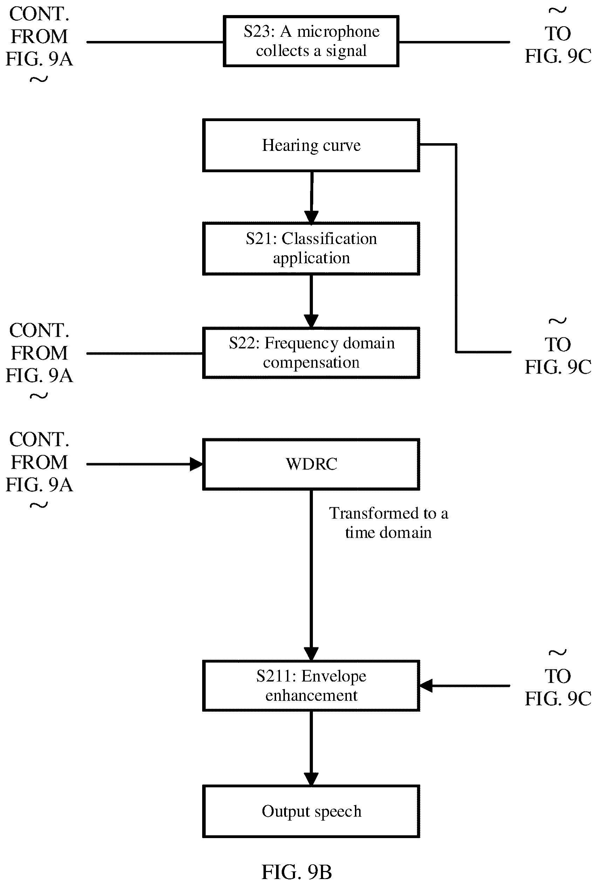

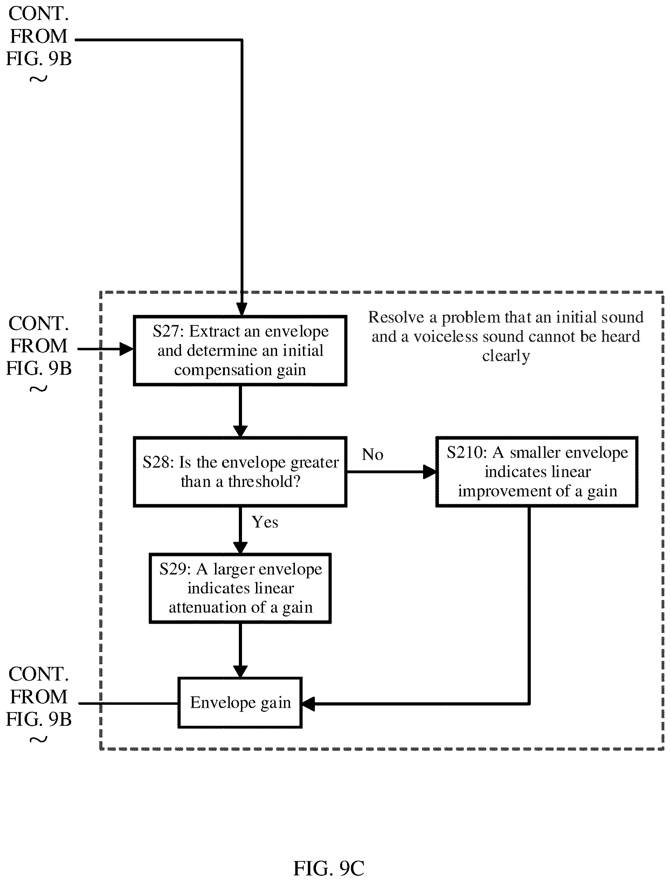

FIG. 9A to FIG. 9C are a functional UI of a compensation procedure. First, a user is indicated to wear a headset by using a picture or the like, and a wearing status is prompted. Then, an audiometry guidance procedure is entered. The user is familiar with an operation by simulating audiometry practice, and more accurate data can be obtained during a normal test. Then, a reaction speed test and volume adjustment are performed to obtain a reaction speed of the user and a most appropriate initial volume value. Finally, audiometry starts to be performed, and hearing chart data of the user is obtained after audiometry is completed.

As shown in FIG. 9A to FIG. 9C, the following describes a compensation procedure mentioned in S5 and S6. The detailed procedure includes the following steps.

S21: Input a hearing curve, and classify hearing curve types based on a predetermined hearing curve library.

S22: Select a corresponding fitting formula based on a classification result, and generate a first frequency domain compensation gain.

S23: A microphone collects an external input signal, and divides the external input signal into a plurality of subbands based on an octave.

S24: Estimate speech signals and noise signals of different subbands, and transmit related information to an intelligibility optimization module such as a speech intelligibility index (speech intelligibility index, SII).

S25: Estimate loudness of hearing loss, and transmit a loudness value to the intelligibility optimization module.

S26: Iteratively optimize a first frequency domain compensation gain based on energy of the speech signals and the noise signals of the different subbands and the loudness of the hearing loss, to obtain a second frequency domain compensation gain that maximizes intelligibility within proper loudness, send the second frequency domain compensation gain to the WDRC for processing, and output a transformed time-domain signal.

S27: Generate an initial first time domain compensation gain based on the hearing curve, and extract an envelope of a microphone input signal.

S28: Determine whether an average value of the envelope is greater than a threshold; for example, detect an average value of several points in the envelope. When the average value is greater than the threshold, linear attenuation of a gain takes effect.

S29: If the envelope is greater than the threshold, a larger envelope indicates linear attenuation of the gain, and a maximum gain is 1, and an envelope gain is output.

S210: If the envelope is less than the threshold, a smaller envelope indicates linear improvement of the gain, and a minimum gain is 1, and an envelope gain is output.

S211: Process an envelope of the time-domain signal based on the envelope gain.

In the foregoing compensation procedure, two methods: frequency domain compensation and time domain compensation are combined. In frequency domain compensation, a dynamic compensation method is introduced based on an environment signal. In time domain compensation, envelope enhancement is used to enhance envelopes of a small signal and a voiceless signal, thereby achieving enhancement with an optimal effect.

Specifically, in terms of frequency domain compensation, an environmental signal-to-noise ratio is estimated in real time, and real-time gain adjustment is performed based on the loudness and the SII, to achieve an optimal fitting effect of intelligibility and loudness.

In terms of time domain compensation, a signal envelope is extracted in real time, and an envelope gain is generated by comparing an envelope amplitude with a threshold, and is applied to the signal, and small signals such as an initial sound and a voiceless sound are amplified additionally, to further improve speech intelligibility.

Through frequency domain compensation in combination with intelligibility and loudness, and real-time envelope time domain enhancement, the fitting effect can be better improved and the speech recognition rate can be enhanced.

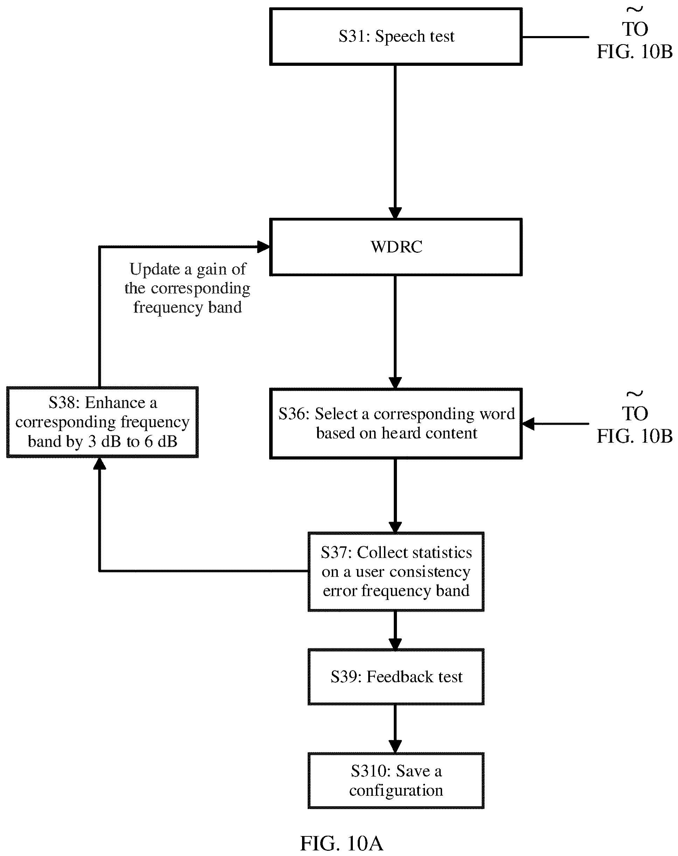

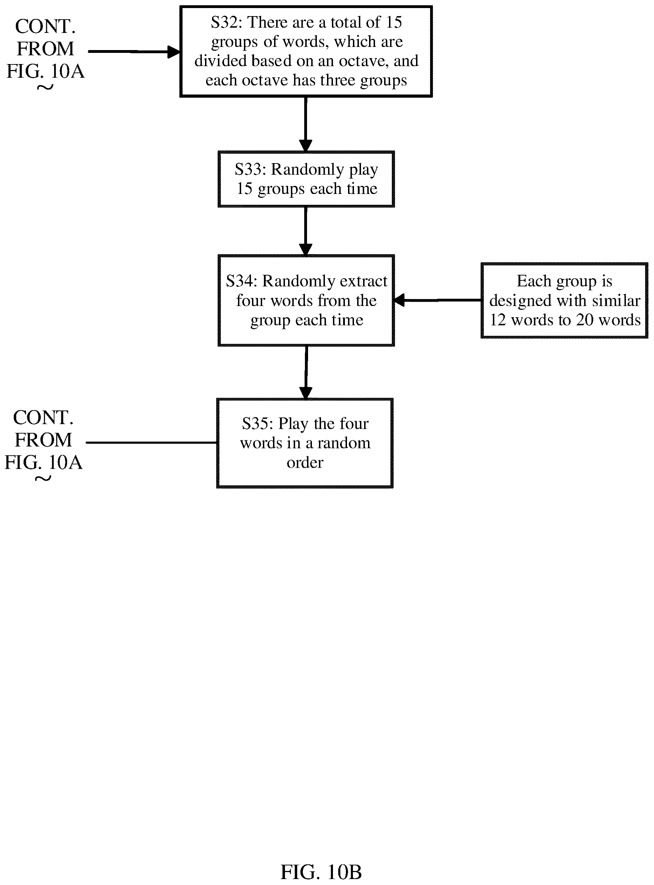

As shown in FIG. 10A and FIG. 10B, the following describes a speech correction and feedback adjustment procedure mentioned in S7 and S8. The detailed procedure includes the following steps.

### S31: Speech test.

S32: There are a total of 15 groups of words, which are divided based on an octave, and each octave has three groups.

S33: Randomly play 15 groups each time.

S34: Randomly extract four words from the group of corresponding words each time.

Each group is designed with similar 12 words to 20 words.

S35: Play the four words in a random order.

S36: Select a corresponding word based on heard content.

In the speech correction procedure, a corpus is provided. The corpus is divided into 15 groups based on an initial consonant-based single-character, a spectral centroid, and an octave. The 15 groups are played in the random order. Each group is designed with similar 12 words to 20 words. Four words are randomly extracted from each group, and the four words are played in the random order. In this way, a problem of speech material reuse effectiveness is resolved.

S37: Collect statistics on a user consistency error frequency band.

S38: Enhance a corresponding frequency band by 3 dB to 6 dB.

A gain of the corresponding frequency band is updated.

Specifically, five groups of feedbacks may be set to collect statistics on the user consistency error frequency band, and the corresponding frequency band is increased by 3 dB to 6 dB based on the consistency error frequency band, so that the WDRC updates the gain of the corresponding frequency band, and differentiated requirements of different users for volumes, timbre equalization, noise reduction, and the like are met in a feedback form of one from two.

For example, a gain condition policy that may be used by the terminal may specifically include: The terminal determines whether a speech volume is appropriate; when the speech volume is too loud, decreases a gain by 4 dB for a frequency of 1000 ... 2000 Hz; when the speech volume is too small, increases a gain by 4 dB for a frequency of 1000 ... 2000 Hz, or increases a gain by 2 dB for 1000 ... 2000 Hz; and when the speech volume is normal, keeps an original gain unchanged and no adjustment is performed.

### S39: Feedback test.

### S310: Save a configuration.

The problem of speech material reuse effectiveness is resolved by randomly selecting a speech material from the corpus, and a compensation gain is fine-tuned based on a speech recognition rate result of the user. In a feedback adjustment process, the user performs feedbacks on the sentence in a plurality of dimensions such as a noise and an overall volume, to perform personalized adjustment on the compensation gain.

In the speech correction module, several words are randomly displayed in the terminal interface, and an audio of one of the words is played. The user selects a heard audio and repeats a plurality of rounds. After the test is completed, the module calculates, based on the speech recognition rate, and applies gains that need to be fine-tuned in different frequency bands. In the feedback adjustment module, the terminal plays a specific sentence, and the user performs selection based on an actual auditory sense. The module performs personalized adjustment such as noise reduction, overall loudness, and a gain based on a selection result of the user.

Through the foregoing fitting procedure performed by the terminal, the user is guided to independently complete a series of fitting steps such as accurate audiometry, real-time time-frequency domain compensation, speech correction, and feedback adjustment, and ensure a good personalized fitting effect.

Embodiments of this application have the following advantages. In terms of audiometry, in embodiments of this application, the tap speed of the user is detected, and the random test signal is generated within the specific range based on the tap speed of the user, to avoid inertia fatigue of the user. In addition, the single-state button avoids the misoperation of the user and the psychological hint. The random mute period is added to the test process, to detect tap accuracy of the user. Iterative determining of the flip point and re-testing of the extreme value point reduce the random error and improve accuracy.

In addition, the fitting formula in embodiments of this application uses a combination of two methods: frequency domain compensation and time domain compensation. In frequency domain compensation, a dynamic compensation method is introduced based on an environment signal. In time domain compensation, envelope enhancement is used to enhance envelopes of a small signal and a voiceless signal, thereby achieving enhancement with an optimal effect. In terms of speech correction, the corpus is designed. The corpus is divided into 15 groups based on an initial consonant-based single-character, a spectral centroid, and an octave. The 15 groups are randomly played. Four words are randomly extracted from each group, and the four words are played in the random order, to resolve a problem of speech material reuse effectiveness. In addition, five groups of feedbacks are set, and differentiated requirements of different users for volumes, timbre equalization, noise reduction, and the like in a feedback form of A/B.

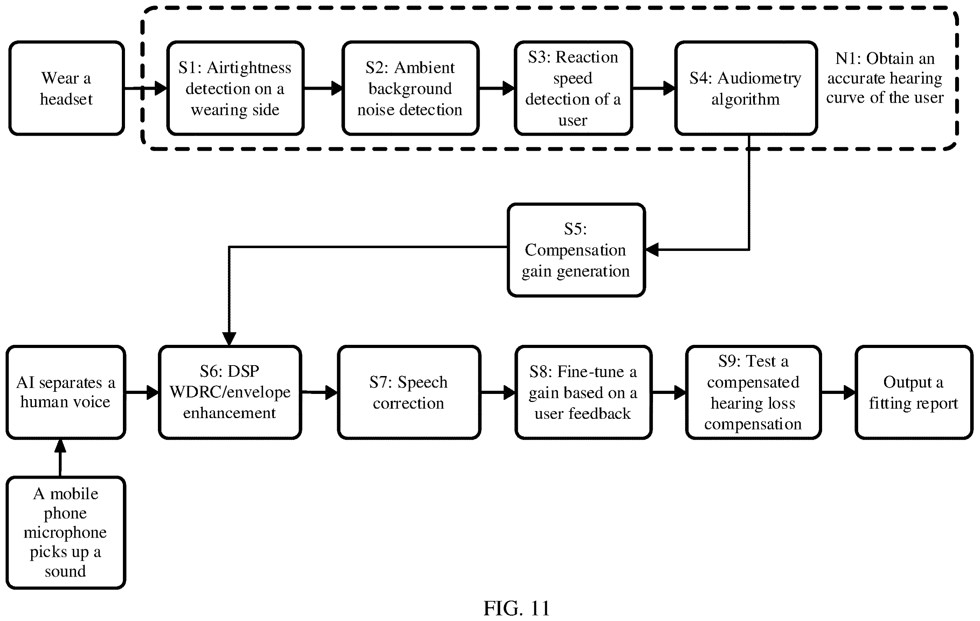

As shown in FIG. 11, in another embodiment provided in this application, compared with that shown in FIG. 6, a terminal performs artificial intelligence (artificial intelligence, AI) separation on a sound pickup signal of a headset microphone in a hearing-assistive enhancement mode, and performs WDRC compensation and envelope enhancement on an extracted human voice, so that a better human voice compensation effect can be provided, and a speech recognition rate can be improved.

As shown in FIG. 11, main steps of the procedure are described as follows:
S1: A user wears a headset, and first detects an FF/FB signal based on a prompt tone, and detects wearing consistency. If wearing is good, perform S2; otherwise, play a prompt tone 1 to the user.
S2: After the wearing consistency is detected, detect an ambient background noise. If the background noise is less than a specific value, perform S3; otherwise, play a prompt tone 2 to the user.
S3: Play a pure-tone test signal to the user, detect a tap speed of the user, and send the tap speed to an audiometry algorithm.
S4: Generate an audiometry test signal based on the tap speed of the user and an initial volume, and play the audiometry test signal to the user. The user taps a feedback button when hearing the test signal. After the test is completed, generate audiometry curves of a left ear and a right ear.
S5: Classify the hearing curves based on an audiometry result, and generate a corresponding frequency domain compensation gain and time domain compensation gain based on a classification result and a corresponding fitting formula.
S6: Deliver the frequency domain compensation gain and the time domain compensation gain to a WDRC module and an envelope modulation module in a DSP of the headset, and amplify an uplink signal and a downlink signal.
S7: Play a preset speech test signal to the user, and fine-tune a compensation gain based on a test result.
S8: Play a specific sentence to the user, and determine a condition such as an optimal listening volume of the user based on a feedback of the user.
S9: Finally, perform a PTA frequency audiometry on the user, and output a fitting report.
S10: Separate a human voice in an environment through AI separation, and send the human voice to S6 for amplification and enhancement.

An AI technology is introduced to separate the human voice from the signal picked up by the microphone of the mobile phone. WDRC frequency-domain compensation and time-domain envelope enhancement are separately performed on the human voice signal to further improve definition of the human voice and a signal-to-noise ratio of the signal, thereby improving the speech recognition rate and achieving a better fitting effect.

Embodiments of this application provide a full-process autonomous fitting procedure, and accurate audiometry, time-frequency domain compensation, and speech correction and feedback adjustment after compensation are performed to maximize alignment with a manual fitting effect.

It should be noted that for ease of brief description, the foregoing method embodiments are represented as a series of action combinations. However, a person skilled in the art should appreciate that this application is not limited to the described order of the actions, and because according to this application, some steps may be performed in other orders or simultaneously. It should be further appreciated by a person skilled in the art that embodiments described in this specification all belong to preferred embodiments, and the related actions and modules are not necessarily required by this application.

To better implement the solutions of embodiments of this application, a related apparatus for implementing the solutions is further provided below.

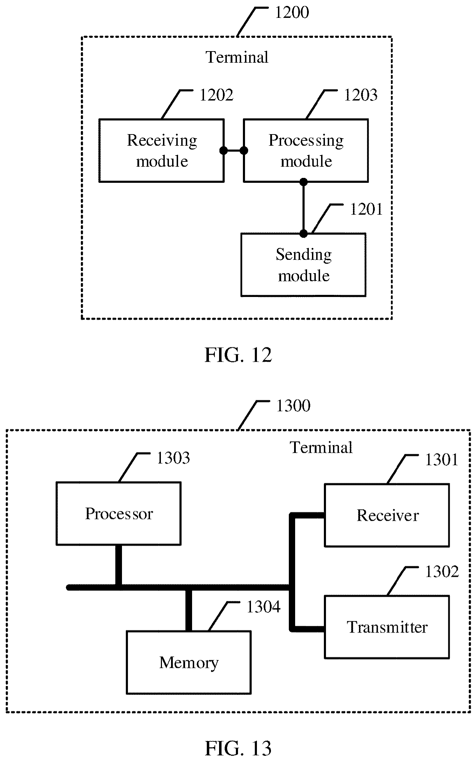

FIG. 12 shows a terminal 1200 provided in an embodiment of this application. A communication connection is established between the terminal and a hearing-assistive device to be fitted. The terminal includes a test module 1201, a compensation module 1202, and a sending module 1203.

The test module is configured to perform a hearing loss test on a hearing-assistive object to obtain a hearing loss test result.

The compensation module is configured to perform hearing loss compensation based on the hearing loss test result to obtain a hearing loss compensation gain.

The sending module is configured to send the hearing loss compensation gain to the hearing-assistive device, where the hearing-assistive device is configured to perform hearing loss compensation on a first speech signal based on the hearing loss compensation gain to obtain a second speech signal.

It should be noted that, because content such as information exchange between the modules/units of the apparatus and the execution processes is based on the same concept as the method embodiments of this application, technical effects brought are the same as those of the method embodiments of this application. For specific content, refer to the descriptions in the foregoing method embodiments of this application. Details are not described herein again.

An embodiment of this application further provides a computer storage medium. The computer storage medium stores a program. The program is executed to perform some or all of the steps recorded in the method embodiments.

The following describes another terminal provided in an embodiment of this application. Refer to FIG. 13. A terminal 1300 includes:
a receiver 1301, a transmitter 1302, a processor 1303, and a memory 1304 (where there may be one or more processors 1303 in the terminal 1300, and an example in which there is one processor is used in FIG. 13). In some embodiments of this application, the receiver 1301, the transmitter 1302, the processor 1303, and the memory 1304 may be connected through a bus or in another manner. In FIG. 13, a connection through a bus is used as an example.

The memory 1304 may include a read-only memory and a random access memory, and provide instructions and data to the processor 1303. A part of the memory 1304 may further include a non-volatile random access memory (non-volatile random access memory, NVRAM). The memory 1304 stores an operating system and operation instructions, an executable module or a data structure, a subset thereof, or an extended set thereof. The operation instructions may include various operation instructions used to implement various operations. The operating system may include various system programs, to implement various basic services and process a hardware-based task.

The processor 1303 controls an operation of the terminal, and the processor 1303 may also be referred to as a central processing unit (central processing unit, CPU). In specific application, components of the terminal are coupled together through a bus system. In addition to a data bus, the bus system may further include a power bus, a control bus, a status signal bus, and the like. However, for clear description, various types of buses in the figure are referred to as the bus system.

The method disclosed in the foregoing embodiments of this application may be applied to the processor 1303, or may be implemented by the processor 1303. The processor 1303 may be an integrated circuit chip and has a signal processing capability. In an implementation process, steps in the foregoing methods can be implemented by using a hardware integrated logic circuit in the processor 1303, or by using instructions in a form of software. The processor 1303 may be a general-purpose processor, a digital signal processor (digital signal processing, DSP), an application-specific integrated circuit (application-specific integrated circuit, ASIC), a field-programmable gate array (field-programmable gate array, FPGA) or another programmable logic device, a discrete gate or transistor logic device, or a discrete hardware component. The processor may implement or perform the methods, the steps, and logical block diagrams that are disclosed in embodiments of this application. The general-purpose processor may be a microprocessor, or the processor may be any conventional processor or the like. The steps of the method disclosed with reference to embodiments of this application may be directly performed by a hardware decoding processor, or may be performed by using a combination of hardware in the decoding processor and a software module. The software module may be located in a mature storage medium in the art, such as a random access memory, a flash memory, a read-only memory, a programmable read-only memory, an electrically erasable programmable memory, or a register. The storage medium is located in the memory 1304, and the processor 1303 reads information in the memory 1304 and completes the steps in the foregoing methods in combination with hardware of the processor.

The receiver 1301 may be configured to: receive entered digital or character information, and generate a signal input related to a related setting and function control of the terminal. The transmitter 1302 may include a display device such as a display, and the transmitter 1302 may be configured to output digital or character information through an external interface.

In this embodiment of this application, the processor 1303 is configured to perform the hearing loss compensation method shown in FIG. 2 or FIG. 3.

In another possible design, when the terminal is a chip in a device, the chip includes a processing unit and a communication unit. The processing unit may be, for example, a processor. The communication unit may be, for example, an input/output interface, a pin, or a circuit. The processing unit may execute computer-executable instructions stored in a storage unit, to enable the chip in the terminal to perform the method according to the first aspect. In some embodiments of this application, the storage unit is a storage unit in the chip, for example, a register or a buffer. Alternatively, the storage unit may be a storage unit in the terminal but outside the chip, for example, a read-only memory (read-only memory, ROM), another type of static storage device that can store static information and instructions, or a random access memory (random access memory, RAM).

The processor mentioned anywhere above may be a general-purpose central processing unit, a microprocessor, an ASIC, or one or more integrated circuits that are configured to control program execution of the method according to the first aspect.

In addition, it should be noted that the described apparatus embodiment is merely an example. The units described as separate parts may or may not be physically separate, and parts displayed as units may or may not be physical units, may be located in one position, or may be distributed on a plurality of network units. Some or all the modules may be selected based on actual needs to achieve the objectives of the solutions of embodiments. In addition, in the accompanying drawings of the apparatus embodiments provided in this application, connection relationships between modules indicate that the modules have communication connections with each other, which may be specifically implemented as one or more communication buses or signal cables.

Based on the descriptions of the foregoing implementations, a person skilled in the art may clearly understand that this application may be implemented by software in addition to necessary universal hardware, or by dedicated hardware, including an application-specific integrated circuit, a dedicated CPU, a dedicated memory, a dedicated component, and the like. Generally, any functions that can be performed by a computer program can be easily implemented through corresponding hardware. Moreover, a specific hardware structure used to achieve a same function may be in various forms, for example, in a form of an analog circuit, a digital circuit, or a dedicated circuit. However, as for this application, software program implementation is a better implementation in most cases. Based on such an understanding, the technical solutions of this application essentially or the part contributing to the conventional technology may be implemented in a form of a software product. The computer software product is stored in a readable storage medium, such as a floppy disk, a USB flash drive, a removable hard disk, a ROM, a RAM, a magnetic disk, or an optical disc of a computer, and includes several instructions for instructing a computer device (which may be a personal computer, a server, a network device, or the like) to perform the methods described in embodiments of this application.

All or some of the foregoing embodiments may be implemented by using software, hardware, firmware, or any combination thereof. When software is used to implement embodiments, all or some of embodiments may be implemented in a form of a computer program product.

The computer program product includes one or more computer instructions. When the computer program instructions are loaded and executed on the computer, the procedure or functions according to embodiments of this application are all or partially generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible to a computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (Solid State Disk, SSD)), or the like.

## Claims

1. A hearing-assistive device-based hearing loss compensation method, wherein the method is performed by a terminal, and a communication connection is established between the terminal and a hearing-assistive device to be fitted, and the method comprises:
performing a hearing loss test on a hearing-assistive object to obtain a hearing loss test result;
performing hearing loss compensation based on the hearing loss test result to obtain a hearing loss compensation gain; and
sending the hearing loss compensation gain to the hearing-assistive device, wherein the hearing-assistive device is configured to perform hearing loss compensation on a first speech signal based on the hearing loss compensation gain to obtain a second speech signal.

2. The method according to claim 1, wherein performing the hearing loss test on the hearing-assistive object to obtain the hearing loss test result comprises:
performing hearing detection on the hearing-assistive object to obtain a feedback speed of the hearing-assistive object;
generating a first test signal based on the feedback speed and a preset first volume, and sending the first test signal to the hearing-assistive device; and
obtaining the hearing loss test result based on the first test signal and a first feedback instruction, wherein the first feedback instruction comprises: a feedback instruction detected by the terminal when the hearing-assistive device plays the first test signal.

3. The method according to claim 2, wherein performing hearing detection on the hearing-assistive object to obtain the feedback speed of the hearing-assistive object comprises:
sending a second test signal to the hearing-assistive device; and
obtaining the feedback speed based on the second test signal and a second feedback instruction, wherein the second feedback instruction comprises: a feedback instruction detected by the terminal when the hearing-assistive device plays the second test signal.

4. The method according to claim 2 or 3, wherein generating the first test signal based on the feedback speed and the preset first volume comprises:
generating the first test signal with a random length based on the feedback speed and the preset first volume, wherein the first test signal comprises test signals at a plurality of frequencies used to measure a hearing loss level of the hearing-assistive object.

5. The method according to any one of claims 2 to 4, wherein the first test signal comprises a random mute signal; and
the method further comprises:
when the hearing-assistive device plays the random mute signal, detecting a misoperation feedback instruction sent by the hearing-assistive object; and
sending a misoperation prompt signal to the hearing-assistive device based on the misoperation feedback instruction.

6. The method according to any one of claims 2 to 5, wherein generating the first test signal based on the feedback speed and the preset first volume, and sending the first test signal to the hearing-assistive device comprises: generating, based on the feedback speed, the preset first volume, and a preset volume increase/decrease difference, a plurality of first test signals corresponding to a first frequency, and sending the plurality of first test signals to the hearing-assistive device, wherein a volume of one first test signal in the plurality of first test signals is the first volume, and a volume of a first test signal other than the one first test signal in the plurality of first test signals is determined based on the first volume and the volume increase/decrease difference; and
obtaining the hearing loss test result based on the first test signal and the first feedback instruction comprises:
determining a first flip point based on a plurality of first feedback instructions corresponding to the plurality of first test signals, wherein the first flip point comprises a time point at which two adjacent first feedback instructions in the plurality of first feedback instructions indicate different listening results;
obtaining a second flip point adjacent to the first flip point; and
when a difference between the first flip point and the second flip point is greater than a preset sound pressure threshold, clearing a frequency test result corresponding to the first flip point; or
when a difference between the first flip point and the second flip point is less than or equal to a preset sound pressure threshold, obtaining the hearing loss test result based on a frequency test result of the first flip point.

7. The method according to claim 6, wherein obtaining the hearing loss test result based on the frequency test result of the first flip point comprises:
when a quantity of times that the frequency test result reaches an extreme value exceeds a times threshold, and/or a quantity of first test signals sent at the first frequency exceeds a quantity threshold, determining the hearing loss test result based on the frequency test result of the first flip point.

8. The method according to any one of claims 1 to 7, wherein performing hearing loss compensation based on the hearing loss test result to obtain the hearing loss compensation gain comprises:
performing real-time compensation processing in time domain and/or frequency domain based on the hearing loss test result to obtain a first compensation gain; and
performing speech correction on the first compensation gain based on a preset speech test signal to obtain the hearing loss compensation gain.

9. The method according to claim 8, wherein performing real-time compensation processing in time domain and/or frequency domain based on the hearing loss test result to obtain the first compensation gain comprises:
obtaining a first hearing loss value at the first frequency in the hearing loss test result;
determining, based on a mapping relationship between a hearing loss value and a correction gain value, a first correction gain value corresponding to the first hearing loss value in time domain and/or frequency domain; and
determining the first compensation gain based on the first compensation gain value.

10. The method according to claim 9, wherein performing real-time compensation processing in time domain and/or frequency domain based on the hearing loss test result to obtain the first compensation gain further comprises:
determining a hearing curve based on the hearing loss test result; and
determining the mapping relationship between the hearing loss value and the correction gain value based on a classification result corresponding to the hearing curve.

11. The method according to claim 9 or 10, wherein determining, based on the mapping relationship between the hearing loss value and the correction gain value, the first correction gain value corresponding to the first hearing loss value in time domain and/or frequency domain comprises:
dividing, based on an octave, an external input signal collected by the hearing-assistive device into a plurality of subbands;
obtaining speech estimation signals and noise estimation signals corresponding to the plurality of subbands;
performing speech intelligibility index analysis based on the speech estimation signals and the noise estimation signals to obtain loudness of hearing loss;
determining a first frequency domain compensation gain of the first hearing loss value in frequency domain based on the mapping relationship between the hearing loss value and the correction gain value; and
performing iterative optimization on the first frequency domain compensation gain based on the speech estimation signals and the noise estimation signals corresponding to the plurality of subbands and the loudness of the hearing loss, to obtain a second frequency domain compensation gain that maximizes intelligibility within preset loudness, wherein the second frequency domain compensation gain belongs to the first correction gain value.

12. The method according to claim 9 or 10, wherein determining, based on the mapping relationship between the hearing loss value and the correction gain value, the first correction gain value corresponding to the first hearing loss value in time domain and/or frequency domain comprises:
determining a first time domain compensation gain of the first hearing loss value in time domain based on the mapping relationship between the hearing loss value and the correction gain value;
extracting envelope information of an external input signal collected by the hearing-assistive device; and
when an average value of the envelope information is greater than a preset envelope threshold, performing linear attenuation processing on the first time domain compensation gain to obtain a second time domain compensation gain, wherein the second time domain compensation gain belongs to the first correction gain value; or
when an average value of the envelope information is less than or equal to a preset envelope threshold, performing linear improvement processing on the first time domain compensation gain to obtain a third time domain compensation gain, wherein the third time domain compensation gain belongs to the first correction gain value.

13. The method according to any one of claims 8 to 12, wherein performing speech correction on the first compensation gain based on the preset speech test signal to obtain the hearing loss compensation gain comprises:
generating a speech test signal based on an audio spectral centroid in a frequency band division manner, wherein the speech test signal comprises test words at a plurality of frequencies corresponding to different audio spectral centroids;
sending the speech test signal to the hearing-assistive device; and
performing speech correction on the first compensation gain based on the speech test signal and a third feedback instruction to obtain the hearing loss compensation gain, wherein the third feedback instruction comprises a feedback instruction detected by the terminal when the hearing-assistive device plays the third test signal.

14. The method according to any one of claims 1 to 13, wherein the method further comprises:
detecting an ambient noise of the hearing-assistive device, and when the ambient noise is greater than or equal to a preset noise threshold, sending a noise prompt signal to the hearing-assistive device; or
when the ambient noise is less than a preset noise threshold, triggering execution of the following step: performing the hearing loss test on the hearing-assistive object to obtain the hearing loss test result.

15. The method according to any one of claims 8 to 12, wherein performing speech correction on the first compensation gain based on the preset speech test signal comprises:
obtaining the speech test signal from a preset corpus, wherein the corpus comprises a plurality of groups of speech test signals, each group of speech test signals comprises initial consonant-based single-character groups divided into a same group based on an audio spectral centroid and different octaves, and one initial consonant-based single-character group comprises two or more randomly extracted confusable words;
playing the two or more confusable words in the same initial consonant-based single-character group in a random order through the hearing-assistive device;
detecting a fourth feedback instruction sent by the hearing-assistive object;
obtaining a consistency error frequency band of the hearing-assistive object based on the fourth feedback instruction and the speech test signal; and
adjusting the first compensation gain based on the consistency error frequency band.

16. The method according to claim 11, wherein determining, based on the mapping relationship between the hearing loss value and the correction gain value, the first correction gain value corresponding to the first hearing loss value in time domain and/or frequency domain further comprises: performing human voice separation on the external input signal collected by the hearing-assistive device, to obtain a human voice signal; and performing wide dynamic range compression compensation and envelope enhancement on the human voice signal to obtain a compensated and enhanced human voice signal; and
dividing, based on the octave, the external input signal collected by the hearing-assistive device into the plurality of subbands comprises: dividing the compensated and enhanced human voice signal into a plurality of subbands based on the octave.

17. A terminal, wherein a communication connection is established between the terminal and a hearing-assistive device to be fitted, and the terminal comprises:
a test module, configured to perform a hearing loss test on a hearing-assistive object to obtain a hearing loss test result;
a compensation module, configured to perform hearing loss compensation based on the hearing loss test result to obtain a hearing loss compensation gain; and
a sending module, configured to send the hearing loss compensation gain to the hearing-assistive device, wherein the hearing-assistive device is configured to perform hearing loss compensation on a first speech signal based on the hearing loss compensation gain to obtain a second speech signal.

18. A terminal, wherein the terminal comprises a processor and a memory, and the processor and the memory communicate with each other;
the memory is configured to store instructions; and
the processor is configured to execute the instructions in the memory, to perform the method according to any one of claims 1 to 16.

19. A computer-readable storage medium, comprising instructions, wherein when the computer-readable storage medium runs on a computer, the computer is enabled to perform the method according to any one of claims 1 to 16.

20. A computer program product comprising instructions, wherein when the computer program product runs on a computer, the computer is enabled to perform the method according to any one of claims 1 to 16.
